(19) Europäisches Patentamt
European Patent Office
Office européen des brevets

(11) EP 1 484 378 A1

(12) **EUROPEAN PATENT APPLICATION**
published in accordance with Art. 158(3) EPC

(43) Date of publication:
**08.12.2004 Bulletin 2004/50**

(21) Application number: **03708512.3**

(22) Date of filing: **07.03.2003**

(51) Int Cl.7: **C09K 5/06**, A61F 7/08, A41D 31/00

(86) International application number:
**PCT/JP2003/002709**

(87) International publication number:
**WO 2003/076547 (18.09.2003 Gazette 2003/38)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.03.2002 JP 2002067489**
**12.03.2002 JP 2002067491**
**25.06.2002 JP 2002184007**

(71) Applicants:
- **IDEMITSU KOSAN CO., LTD.**
  **Tokyo 100-8321 (JP)**
- **Idemitsu Technofine Co. Ltd**
  **Sumida-ku, Tokyo 130-0015 (JP)**

(72) Inventors:
- **SANO, Masahiro**
  **Sodegaura-shi, Chiba 299-0293 (JP)**
- **ABE, Kazuaki**
  **Sodegaura-shi, Chiba 299-0293 (JP)**
- **MACHIDA, Yoshinori**
  **Sodegaura-shi, Chiba 299-0293 (JP)**
- **UBARA, Atsuhiko**
  **Sodegaura-shi, Chiba 299-0293 (JP)**
- **TAGUCHI, Toshiharu**
  **Sodegaura-shi, Chiba 299-0293 (JP)**

(74) Representative:
**Gille Hrabal Struck Neidlein Prop Roos**
**Patentanwälte,**
**Brucknerstrasse 20**
**40593 Düsseldorf (DE)**

# (54) HEAT STORING MATERIAL, COMPOSITION THEREOF AND THEIR USE

(57) A heat-accumulative material composed of a polymer or oligomer having a melting point of -10 to 100°C and latent heat of 30 J/g or more for use around a body, and heat-accumulative composition comprising the same heat-accumulative material. The heat-accumulative material tends to be kept at a constant temperature more effectively by absorbing heat as ambient temperature increases to melt, and releasing heat as ambient temperature decreases to solidify, to moderate the effect of changed ambient temperature, and thereby to exhibit the function as a heat-accumulative material. They have a sufficiently high viscosity, preventing the heat-accumulative material from flowing out even when it is molten. Each of the heat-accumulative material and composition can be made into a heat-accumulative film or sheet, laminate, molded article, composite fiber and cloth which can be suitably used around a body.

Fig. 1

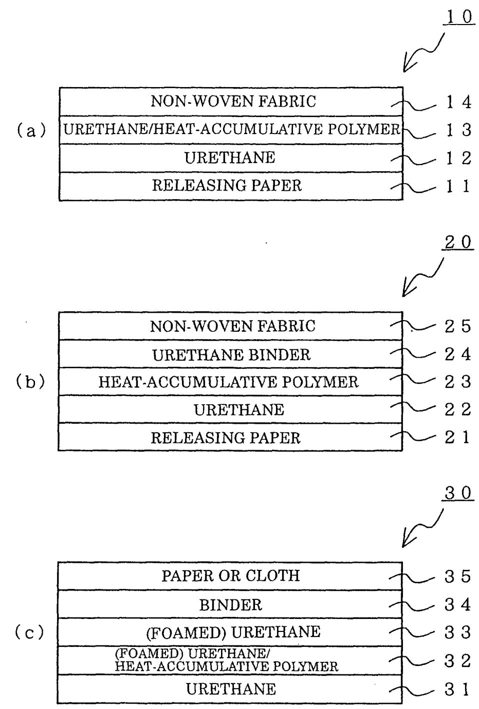


EP 1 484 378 A1

## Description

Technical Field

The present invention relates to a heat-accumulative material, a composition thereof, and a heat-accumulative film or sheet, laminate, composite fiber, cloth and molded article of the above material or composition.

Background Art

Heretofore, clothes worn in an atmosphere of widely varying temperatures, e.g., clothes for cold weather or sporting, have been made of various materials for improving heat insulation.

Examples of the clothes developed so far include clothes using various cotton materials or feathers as heat-insulating materials, clothes in which a radiant heat reflecting film such as a aluminum film is introduced, and clothes made of materials which generate heat on absorbing moisture.

Furthermore, to provide a temperature-controlling function for the change of ambient temperatures, heat-accumulative materials have been used.

An example of such heat-accumulative materials is a low-molecular weight crystallizable compound such as octadecane, and its phase change heat (fusion or solidification) is utilized to adjust the temperature.

Such a compound, although having a large latent heat, has a sufficiently reduced viscosity and increased fluidity, when molten, to cause problems of leakage or spillage. Another problem involved in the compound is evaporation resulting from its low molecular weight and hence a low boiling point, when the compound is processed at an elevated temperature.

Attempts have been made to seal the low-molecular-weight compound in microcapsules, as disclosed in, e.g., Japanese Patent Laid-open Publication Nos. 58-55699, 1-85374 and 2-182980. More specifically, the microcapsules containing a low-molecular-weight compound are spread and fixed on a cloth, or a synthetic resin containing the microcapsules is spun into fibers, which are woven into the cloth. These products have been already realized.

However, the techniques which use these microcapsules involve the following problems:

(1) The microcapsules cannot exhibit their function, because of difficulty in uniformly attaching them to the base material.
(2) The microcapsules, although capable of improving heat insulation, may damage comfortableness of the clothes, when put thereon via an adhesive agent, because of possible adverse effects of the binder on their moisture retention.
(3) The microcapsules, having structurally a certain size, are difficult to form into a thin film, when they are made into a film or sheet.
(4) The microcapsule material is already colored.
(5) The microcapsules may give off formaldehyde.
(6) The microcapsules have poor fabricability, when to be made into a film or sheet, because they may be broken by pressure or the like, and will cause, when broken, leakage of the molten liquid which they hold.
(7) An adhesive agent used for fixing the microcapsules to cloth may harden the cloth, or deteriorate its texture or moisture-permeability required for cloth, with the result that the clothes thereof will have deteriorated functions.
(8) Grain size of the microcapsules is large to possibly cause problems, e.g., yarn cutting during the spinning or weaving process.

On the other hand, heat-accumulative polymers which utilize phase change of the main chain have been developed, as disclosed by, e.g., Japanese Patent Laid-open Publication Nos. 57-76078 and 58-277773.

However, the heat-accumulative polymers falling into this category are too high in melting point to be practical. For example, high-density polyethylene has a melting point of 110 to 130°C. Moreover, it is difficult to control their melting point. Still more, these polymers become fluid at temperature above their melting point, to collapse the molded article thereof.

Japanese Patent Laid-open Publication No. 8-311716 proposes a composite fiber with a core made of a composition of paraffin wax and polyethylene resin as heat-accumulative materials.

However, the composite fiber with the core of paraffin wax composition causes production-related problems resulting from scattering of the wax under heating during the wax incorporation or composite fiber production process, with the result that it may not sufficiently exhibit its heat-accumulative capacity.

Such heat accumulative materials are used for various proposes at present.

Japanese Patent Laid-open Publication No. 5-214328, for example, discloses a heat-accumulative material of alpha-olefin of 18 to 28 carbon atoms, describing an energy-saving type heating system in which heat of solidification

of the heat-accumulative material is utilized as one of the applications.

Japanese Patent Laid-open Publication No. 8-224754 proposes heat-insulated tableware, produced by multilayer injection molding, with a heat-accumulative material contained in a microcapsule of fluorine or silicone resin.

Japanese Patent Laid-open Publication No. 9-174471 proposes a composite heat-insulating panel which incorporates a heat-accumulative material showing phase change in a temperature range from 0 to 30°C for preventing condensation of moisture in air.

More recently, Japanese Patent Laid-open Publication No. 2002-114553 proposes a cement-based building material of heat-accumulative structure which incorporates microcapsules of a latent heat accumulative material.

Japanese Patent No. 3,306,482 proposes a heat exchanger or the like which comprises powdered paraffin wax as latent heat accumulative material.

Japanese Patent Laid-open Publication No. 2002-211967 proposes a temperature- and humidity-controlling material containing microcapsules which hold a latent heat accumulative material, and temperature- and humidity-controlling foam which incorporates the material.

However, the techniques disclosed by the above publications involve following disadvantages.

(1) A common plastic molding system, e.g., injection, blow or compression molding system, may not effectively give a desired product, because it can collapse the microcapsules under pressure.
(2) When a paraffin-based material is used, the product may not effectively exhibit heat-accumulative effect, because of evaporation of the heat-accumulative component during the molding process.
(3) The heat-accumulative material is too high in phase-change temperature to be practical.

Disclosure of the Invention

It is an object of the present invention to provide a heat-accumulative material, a heat-accumulative composition, and heat-accumulative film or sheet, laminate, composite fiber, cloth and molded article using the above material and composition, which are easily fabricated and excellent in heat-accumulative capacity, in consideration of the above problems involved in the conventional techniques.

The present invention provide the following heat-accumulative materials and the like.

[1] A heat-accumulative material for use around a body, comprising a polymer or oligomer whose melting point is from -10°C to 100°C and latent heat is at least 30 J/g.

A polymer or oligomer is structurally not limited, and may be of straight chain, side chain, branched or three-dimensional network.

[2] The heat-accumulative material according to [1], wherein the melting point is from -10°C to 80°C.
[3] The heat-accumulative material according to [1] or [2], wherein difference between the melting point and a solidifying point of the polymer or oligomer is at most 15°C.
[4] The heat-accumulative material according to any one of [1] to [3], wherein the melting point is from 0°C to 50°C.
[5] The heat-accumulative material according to [4], wherein viscosity of a 40 wt.% toluene solution of the polymer or oligomer at 50°C is at least 100 $mm^2/s$.
[6] The heat-accumulative material according to any one of [1] to [5], wherein the latent heat is at least 50 J/g.
[7] A heat-accumulative material comprising, consisting essentially of, or consisting of crystalline polymeric units of formula (1), the unit comprising a main chain section X, a bonding section Y and a side chain Z, side chains Z capable of being crystallized.

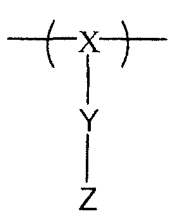

(1)

[8] A heat-accumulative material which is a cross-linked polymer or oligomer comprising, consisting essentially of, or consisting of crystalline units of formula (1), the unit comprising a main chain section X, a bonding section Y and a side chain Z, side chains Z capable of being crystallized.

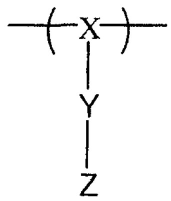

(1)

[9] The heat-accumulative material according to [7] or [8], wherein weights of the main chain section X, the bonding section Y and side chain Z satisfy the following formula:

$$Z / (X + Y + Z) \geqq 0.75.$$

[10] The heat-accumulative material according to any one of [7] to [9], wherein the main chain section X is at least one selected from the group of

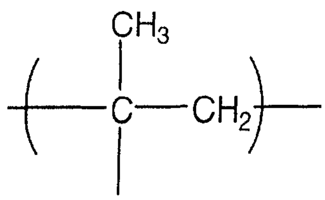 and 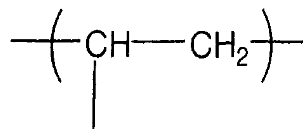 ,

the bonding section and side chain, Y-Z, is at least one selected from the group of -CO-O-R, -O-CO-R, -O-R and $-CH_2$-R, and

R is a hydrocarbon group having 9 or more carbon atoms.

[11] The heat-accumulative material according to any one of [7] to [10], wherein R is a straight hydrocarbon group having 9 or more carbon atoms.

[12] The heat-accumulative material according to [7], which is polydocosyl methacrylate, polyheneicosyl methacrylate, polyeicosyl acrylate, polynonadesyl acrylate, polyheptadesyl acrylate, polypalmityl acrylate, polypentadesyl acrylate, polystearyl acrylate, polylauryl acrylate, polymyristyl acrylate, polymyristyl methacrylate, polypentadesyl methacrylate, polypalmityl methacrylate, polyheptadesyl methacrylate, polynonadesyl methacrylate, polyeicosyl methacrylate, polystearyl methacrylate, poly(palmityl/stearyl) methacrylate, polyvinyl laurate, polyvinyl myristate, polyvinyl palmitate, polyvinyl stearate, polylauryl vinyl ether, polymyristyl vinyl ether, polypalmityl vinyl ether or polystearyl vinyl ether.

[13] The heat-accumulative material according to any one of [7] to [12], the polymer or oligomer further comprising hydrophilic units.

[14] The heat-accumulative material according to [13], the hydrophilic unit is represented by formula (2).

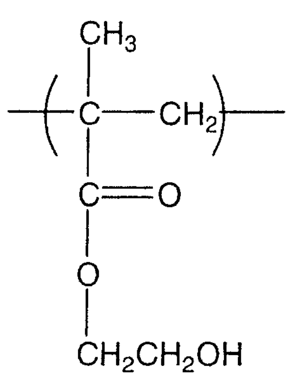

(2)

[15] The heat-accumulative material according to any one of [8] to [14], wherein a cross linking agent is 0.1 to 20

wt.% of monomers forming the crystalline units, or the crystalline units and the hydrophilic units.
[16] A heat-accumulative composition comprising the heat-accumulative material of any one of [1] to [15] and a synthetic resin.
[17] The heat-accumulative composition according to [16], wherein the synthetic resin is at least one selected from the group of polyurethane, acrylic, polyamide, polyvinyl chloride, polypropylene, polyethylene, polystyrene, polyester, polycarbonate, ethylene/vinyl alcohol copolymer, thermoplastic elastomer, polyphenylene sulfide and ABS resins.
[18] A heat-accumulative film or sheet comprising the material of any one of [1] to [15]; or the composition of [16].
[19] A heat-accumulative laminate comprising the film or sheet of [18] as one layer.
[20] A heat-accumulative composite fiber comprising a core and a sheath;
the core comprising the material of any one of [1] to [15]; or the composition of [16] or [17];
the sheath comprising a synthetic resin.
[21] The heat-accumulative composite fiber according to [20], wherein the synthetic resin is at least one selected from the group of polyamide, polyester, polyurethane, ethylene/vinyl acetate copolymer, polyvinylidene chloride, polyvinyl chloride, acrylic, polyethylene and polypropylene resins.
[22] A heat-accumulative cloth comprising the composite fiber of [20] or [21].
[23] A heat-accumulative molded article comprising the material of any one of [1] to [15]; or the composition of [16] or [17].
[24] The heat-accumulative molded article according to [23], which is an energy-saving part or a part for preventing excessive heating or cooling.
[25] The heat-accumulative molded article according to [23], which is a building material, residential good, automobile part, electric/electronic appliance part, heat-exchanger part or heat transfer device part.

Brief Description of the Drawings

Fig. 1 (a), (b) and (c) schematically illustrate the laminate prepared in EXAMPLE 76 and 77, and a variation of the laminate, respectively.
Fig. 2 presents a graph showing temperature changes inside of the laminates prepared in EXAMPLES 76 and 77 and COMPARATIVE EXAMPLE 3, which were exposed to changing environmental temperature.
Fig. 3 presents a graph showing the results of evaluating heat-accumulative capacity of a 4-mm-thick injection-molded plate of the heat-accumulative polypropylene composition prepared in EXAMPLE 96 and polypropylene prepared in COMPARATIVE EXAMPLE 7.
Fig. 4 presents a graph showing temperature-controlling effect of the containers of the heat-accumulative polypropylene composition prepared in EXAMPLE 96 and polypropylene prepared in COMPARATIVE EXAMPLE 7 in the air layer.
Fig. 5 presents a graph showing temperature-controlling effect of the containers of the heat-accumulative polypropylene composition prepared in EXAMPLE 96 and polypropylene prepared in COMPARATIVE EXAMPLE 7 in the water layer.

Best Mode for Carrying out the Invention

The present invention is described in detail below.

[Heat-accumulative material]

The heat-accumulative material of the present invention is (A) or (B) described below:

(A) A polymer or oligomer having, as the main constituent component, a crystalline unit represented by the formula (1), wherein X is a main chain section, Y is a bonding section and Z is a crystallizable side chain.

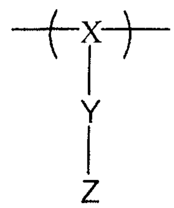

(1)

(B) A cross-linked polymer or oligomer (cross-linked heat-accumulative material) having, as the main constituent component, a crystalline unit represented by the formula (1), wherein X is a main chain section, Y is a bonding section and Z is a crystallizable side chain.

Each of these heat-accumulative materials undergoes phase changes (melting and solidification) in a given temperature range as the side chain Z transforms itself into or from the crystal phase, which is accompanied by releasing or absorbing a large latent heat. Each of these heat-accumulative materials absorbs heat as ambient temperature increases to melt, and releases heat as ambient temperature decreases to solidify. Therefore, these materials can moderate the effect of changed ambient temperature so that it tends to be kept at a constant temperature, thereby exhibiting their function as heat-accumulative materials. In these materials, the main chain section X in the formula (1) does not melt in the above temperature range. Moreover, the material (B) is cross-linked into a three-dimensional network structure. Therefore, they can retain their original shape without running out as a whole. Their melting point can be easily controlled by adjusting length of their side chain.

The main chain section X in the formula (1) is structurally not limited, so long as it does not retard crystallization of the side chain Z. However, it is preferably at least one type selected from the following structures:

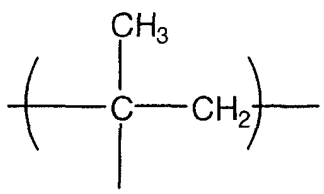

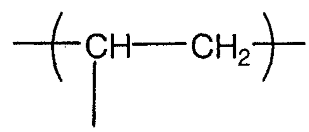

The bonding section Y works to bond the main chain section X and side chain Z to each other, and means one-atom unit. It is preferably at least one type selected from -CO-, -O- and -$CH_2$-.

The side chain Z is not limited, so long as it can be crystallized. However, it preferably contains a hydrocarbon group of 9 or more carbon atoms, more preferably straight-chain alkyl group of 9 or more carbon atoms.

The bonding section and side chain Y-Z is preferably at least one selected from -CO-O-R, -O-CO-R, -O-R and - $CH_2$-R wherein R is preferably a hydrocarbon group of 9 or more carbon atoms, more preferably straight-chain alkyl group of 9 or more carbon atoms.

The particularly preferable units composed of the main chain section X, bonding section Y and side chain Z are polymethacrylate-, polyacrylate, polyvinyl ester-, polyvinyl ether- and hydrocarbon-based ones, described below.

(Methacrylate-Based Unit)

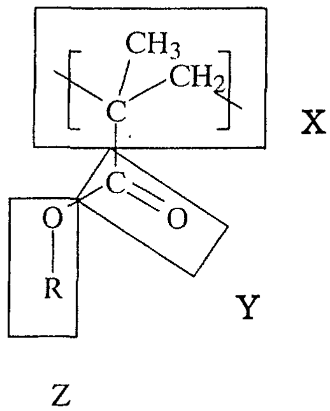

R: straight-chain alkyl group of 14 or more carbon atoms

(Acrylate-Based Unit)

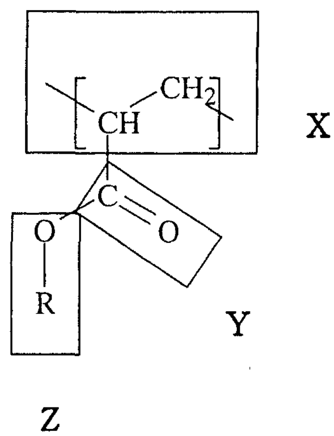

R: straight-chain alkyl group of 12 or more carbon atoms

(Vinyl Ester-Based Unit)

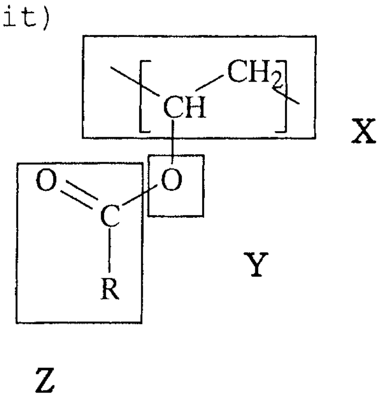

R: straight-chain alkyl group of 9 or more carbon atoms

(Vinyl Ether-Based Unit)

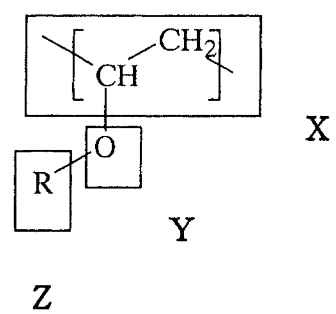

R: straight-chain alkyl group of 10 or more carbon atoms

(Hydrocarbon-Based Unit)

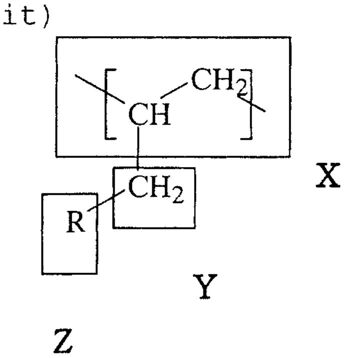

R: straight-chain alkyl group of 9 or more carbon atoms

R for each unit satisfies the following relationship:

$$A = Z/(X+Y+Z) \geq 0.75$$

Examples of the preferable heat-accumulative material (A) include long-chain alkyl hydrocarbon esters of methacrylic or acrylic acid. More specifically, they include polydocosyl methacrylate, polyheneicosyl methacrylate, polyeicosyl acrylate, polynonadesyl acrylate, polyheptadesyl acrylate, polypalmityl acrylate, polypentadesyl acrylate, polystearyl acrylate, polylauryl acrylate, polymyristyl acrylate, polymyristyl methacrylate, polypentadesyl methacrylate, polypalmityl methacrylate, polyheptadesyl methacrylate, polynonadesyl methacrylate, polyeicosyl methacrylate, polystearyl methacrylate, poly(palmityl/stearyl) methacrylate, polyvinyl laurate, polyvinyl myristate, polyvinyl palmitate, polyvinyl stearate, polylauryl vinyl ether, polymyristyl vinyl ether, polypalmityl vinyl ether and polystearyl vinyl ether.
Examples of the preferable and more preferable heat-accumulative material (B) include the cross-linked compounds cited as the preferable and more preferable ones for the heat-accumulative material (A).
Contents by weight of the main chain section X, bonding section Y and side chain Z preferably satisfies the following relationship:

$$A = Z/(X+Y+Z) \geq 0.75$$

In other words, the side chain Z accounts for 75% by weight or more of the crystallizable unit. A heat-accumulative material containing the side chain Z at less than 75% by weight may not be crystallized to exhibit heat-accumulative capacity.
The heat-accumulative material (A) or (B) may be incorporated with another unit to exhibit a desired function, so long as it is not harmful to the characteristics of the heat-accumulative material.
For example, the heat-accumulative material (A) or (B) may be incorporated with a hydrophilic unit. These heat-accumulative materials, containing a long-chain hydrocarbon group as the side chain, are highly hydrophobic. However, they can have enhanced hydrophilicity when incorporated with a hydrophilic unit, and hence enhanced adhesion to a base or the like, when applied thereon.
The monomer for forming the hydrophilic unit is not limited. However, the preferable monomers include 2-hydroxyethyl acrylate and 2-hydroxyethyl methacrylate, the latter being more preferable. The hydrophilic unit comprising 2-hydroxyethyl methacrylate is represented by the formula (2):

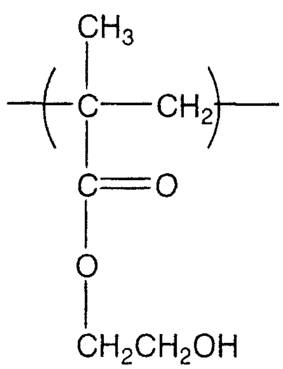

(2)

The hydrophilic unit is present preferably at 50% by weight or less, more preferably 30% by weight or less. The side chain Z may have deteriorated crystallizability, when it is present at above 50% by weight.
The heat-accumulative materials (A) and (B) preferably have a weight-average molecular weight Mw of 1,000 to 2,000,000, more preferably 10,000 to 1,000,000. A heat-accumulative material having an Mw value below 1,000 may give a defective product, which is insufficient in strength, and may be liquefied while in use and become sticky while in use, because of its low melting point. On the other hand, a heat-accumulative material having an Mw value above 2,000,000 may be deteriorated in spinning characteristics and moldability, because of its insufficient fluidity as a polymer.
The heat-accumulative materials (A) and (B) preferably have a melting point, at which the side chain is transformed from the crystal state, of -10 to 100°C. The more preferable lower limit of the range is 0°C, still more preferably 10°C. The more preferable higher limit of the range is 80°C, still more preferably 50°C.
A heat-accumulative material having a melting point above 100°C is always present as solid in the common service atmosphere, and may be difficult to fully exhibit its heat-accumulative function, because absorption of the crystallization heat is no longer expected while it is being heated.
On the other hand, a heat-accumulative material having a melting point below -10°C is always present as liquid in the common service atmosphere, and may be difficult to fully exhibit its heat-accumulative function, because

releasing heat is no longer expected while it is being solidified.

The difference between melting point and freezing point of the heat-accumulative materials (A) and (B) is preferably within 15°C. When the difference is beyond 15°C, the range in which they absorb and release heat will be too wide for these materials to fully exhibit their heat-accumulative function in a desired narrow temperature range.

The heat-accumulative materials (A) and (B) preferably have a latent heat of 30 J/g or more, more preferably 50 J/g or more, still more preferably 70 J/g or more. A heat-accumulative material having a latent heat below 30 J/g may exhibit a heat-accumulative function insufficiently. Normally, it is 200 J/g or less.

The side chain Z in the heat-accumulative material (A) or (B) reversibly transforms itself into or from the crystal state in a given temperature range, while releasing or absorbing a large latent heat. The main chain X, on the other hand, shows no such phase transformation.

The 40% by weight toluene solution of the heat-accumulative material (A) or (B) preferably has a solution viscosity of 100 $mm^2/s$ or more at 50°C, more preferably 120 $mm^2/s$ or more. A heat-accumulative material having the viscosity below 100 $mm^2/s$ may leak out of the cloth in which it has been incorporated to cause troubles, e.g., making the cloth sticky.

Melting point, freezing point and latent heat are measured by differential scanning calorimetry (DSC). Melting point and freezing point mean the melting and crystallization peak temperature, respectively (JIS K-7121). Melting point is defined as the melting peak temperature, observed when the sample is heated beyond the melting point once, then cooled to a given temperature and reheated.

The process for producing the heat-accumulative material (A) or (B) is not limited.

For example, the heat-accumulative material (A) can be produced by polymerizing a monomer capable of forming the crystallizable unit, or monomers capable of forming the crystallizable and hydrophilic units.

The heat-accumulative material (B) can be produced by polymerizing a monomer capable of forming the crystallizable unit, or monomers capable of forming the crystallizable and hydrophilic units together with a cross-linking agent.

The cross-linking agents (monomers) useful for the present invention to form cross-linking include polyethylene glycol (1000) diacrylate, polyethylene glycol (1000) dimethacrylate, ethylene glycol diacrylate and ethylene glycol dimethacrylate, and preferable are polyethylene glycol (1000) dimethacrylate and ethylene glycol dimethacrylate.

Content of the cross-linking agent is preferably 0.1 to 20% by weight on the monomer for forming the crystallizable and hydrophilic units, more preferably 0.2 to 3% by weight. When the content is less than 0.1% by weight, a cross-linking effect can scarcely be exerted, while when it is beyond 20% by weight, an additional effect can scarcely be expected.

The heat-accumulative material of the present invention is very useful as a heat-accumulative material, because it tends to be kept at a constant temperature more effectively by absorbing heat as ambient temperature increases to melt, and releasing heat as ambient temperature decreases to solidify, to moderate the effect of changed ambient temperature.

Each of the heat-accumulative materials (A) and (B) can exhibit the following effects, in addition to the above.

(1) It is sufficiently high in molecular weight not to cause evaporation or leakage.
(2) It is a resin, and easily processable. It can be applied, kneaded into another material or made into fibers.
(3) Its melting point can be easily controlled by controlling length of the side chain Z.
(4) It can have another function, when copolymerized.
(5) It will not ooze out during the melting process to retain its original shape.
(6) It shows a sharper phase change than that of materials whose main chain is crystallized to exhibit heat accumulative properties.
(7) The heat-accumulative material (B) is cross-linked into a moldable thermoplastic resin.
(8) The heat-accumulative material (B) is cross-linked to give a molded article capable of retaining its original shape even at above melting point.

[Heat-accumulative composition]

The heat-accumulative composition of the present invention comprises the above heat-accumulative material incorporated in a resin (synthetic resin).

The synthetic resin for the heat-accumulative composition preferably has a melting point of 100°C or higher. More specifically, those resins useful for the present invention include polyurethane, acrylic, polyamide, polyvinyl chloride (PVC), polypropylene, polyethylene, polystyrene, polyester (e.g., PET), polycarbonate, ethylene/vinyl alcohol copolymer, thermoplastic elastomer, polyphenylene sulfide and ABS. They may be used either individually or in combination.

Content of the heat-accumulative material varies depending on the required temperature-controlling function.

However, it is preferably 5% by weight or more of the synthetic resin, more preferably 20% by weight or more, still more preferably 30% by weight or more.
It may not fully exhibit its temperature-controlling function at below 5% by weight. At above 90% by weight, on the other hand, the base material may be easily hardened and turn fragile.
The heat-accumulative composition may contain an epoxy-containing acrylic polymer, allyl ether copolymer or the like as a compatibility improver. The improver can make the synthetic resin more compatible and allow to increase content of the heat-accumulative material.
Moreover, the heat-accumulative composition may contain one or more various additives so far as an additive does not harm its characteristics. The additives useful for the present invention include antioxidant, light-resistance improver, inorganic filler (e.g., calcium carbonate or talc), foaming agent (e.g., chemical foaming agent), aging inhibitor, antimicrobial agent, antifungal agent, colorant, pigment, antistatic agent, flame retardant, processing aid, stabilizer, plasticizer, cross-linking agent and reaction promoter.
The heat-accumulative composition preferably has a latent heat of 1 J/g or more in a temperature range of -10 to 100°C for its heat-accumulative function, more preferably 5 J/g or more. A heat-accumulative composition having a latent heat below 1 J/g may not fully exhibit the heat-accumulative effect. Its latent heat is still more preferably 1 J/g or more, still more preferably 5 J/g or more, preferably in a temperature range of -10 to 80°C, more preferably 0 to 50°C.
These characteristics allow the heat-accumulative composition to fully exhibit its temperature-controlling function against ambient temperature or the like.
The heat-accumulative composition may be produced by blending/kneading the heat-accumulative material and synthetic resin by a known process.
The heat-accumulative material or composition of the present invention can be suitably used around the body of a human or animal with its excellent heat-accumulative capacity, where "around the body of a human or animal" means that it may be or may not be in direct contact with the object. The heat-accumulative material of the present invention can control temperature around the body in association with body temperature.
More specifically, the heat-accumulative material and composition can be suitably used for sporting clothes (e.g., skiing wear and rain wear), winter clothes, common clothes (e.g., stockings, panty stockings, shirts and suits), bedclothes and beds (e.g., cotton thereinside), gloves, shoes, furniture, artificial leather for automobiles, food packing materials which need high- or low-temperature insulation, and building materials. When used for furniture or leather for automobiles, they can moderate rate of temperature increase by body temperature at the portion coming into direct contact with the person, making him more comfortable in summer.

[Film or sheet]

The film or sheet of the present invention is composed of the heat-accumulative material or composition described above.
The method for forming the heat-accumulative material or composition into film or sheet is not limited, and may be selected from the known ones. More specifically, the methods include knife coating, gravure coating, spraying and dipping.
The heat-accumulative material or composition may be molded by the method for thermoplastic resins, e.g., common T-die, inflation, compression or calendaring molding, in particular when the composition contains polyvinyl chloride, polyamide, polypropylene, polyethylene, polystyrene or polyester resin.
Moreover, the heat-accumulative composition containing polyurethane, acrylic or polyamide resin can be made into film after being dissolved in a solvent. The solvents useful for producing the resin solution include dimethylformamide, methylethylketone and toluene.
The heat-accumulative material may be made into film after being finely powdered with a varying resin and then emulsified in a poor solvent, e.g., water or isopropyl alcohol.
The film or sheet of the present invention can be produced without causing evaporation or leakage, because the heat-accumulative material or composition as the starting material has a sufficiently high molecular weight. The starting material is a resin, and easily processable. It can be applied, kneaded into another material or made into fibers. In other words, the heat-accumulative material or composition can be easily made into film or sheet, in which it can be dispersed more uniformly than that produced by the conventional technique.
The heat-accumulative material may contain one or more various additives described above so far as an additive does not harm its characteristics.
The film or sheet of the present invention, containing the heat-accumulative material, generates its latent heat when the heat-accumulative material melts. More specifically, it generates a latent heat preferably of 1 J/g or more, more preferably 5 J/g or more. If its latent heat is less than 1 J/g, it may not exhibit sufficient heat accumulative effect. Its latent heat is more preferably 1 J/g or more, still more preferably 5 J/g or more, more preferably in a temperature

range of -10 to 80°C, still more preferably 0 to 50°C.

[Laminate]

The laminate of the present invention has a multi-layered structure with 2 or more layers, one layer of which is of the above film or sheet.

The laminate of the present invention is preferably produced by laminating the film or sheet on a base. The bases useful for the present invention include polyvinyl chloride (PVC) sheet, polyurethane sheet, fibrous cloth, cellulose, synthetic resin film (e.g., polyester or polypropylene film), non-woven fabric and paper.

The laminate of the present invention may contain, in addition to the film or sheet of the present invention and base, a binder layer, as required, between the base and film layer.

The process for producing the laminate of the present invention is not limited, and may be selected from the known ones. More specifically, the processes include knife coating, gravure coating, spraying and dipping.

Moreover, the laminate may be produced by the molding processes used for thermoplastic resins, e.g., common T-die, inflation, compression or calendaring molding, when the heat-accumulative composition contains polyvinyl chloride, polyamide, polypropylene, polyethylene, polystyrene, polyester resin or the like.

The laminate of the present invention may be also produced by putting the film or sheet of the present invention on another layer via a binder or the like.

The present invention can provide an excellent heat-accumulative film or sheet, and laminate less sensitive to ambient temperature changes, because it comprises the material or its composition high in heat-accumulative capacity.

The heat-accumulative film or sheet, and laminate of the present invention can find uses similar to those for the heat-accumulative material. They are particularly suitable for textiles, furniture and artificial leather for automobiles, among others.

For example, when a person who wears a textile product of the fiber cloth on which the film or sheet of the present invention is laminated enters a hot space, the polymer absorbs the latent heat to prevent temperature rise of his clothes thereby protecting him more efficiently from the effect of ambient temperature. When he enters a cold space, the polymer generates the solidification heat to solidify and thereby to prevent temperature decrease of his clothes. Therefore, the heat-accumulative film or sheet, and laminate of the present invention can be used for the so-called textile product of temperature-controlling function, e.g., wear for cold weather or sporting.

Moreover, when used for furniture or leather for automobiles, they can moderate rate of temperature increase by human temperature at the portion coming into direct contact with the person, making him more comfortable in summer, as is the case with the heat-accumulative material.

[Heat-accumulative composite fiber]

The heat-accumulative composite fiber of the present invention has a core/sheath structure, with the heat-accumulative material or composition for the core and a synthetic resin for the sheath.

The synthetic resins useful for the sheath of the heat-accumulative composite fiber of the present invention include polyamide, polyester, polyurethane, ethylene/vinyl acetate copolymer, polyvinylidene chloride, polyvinyl chloride, acrylic, polyethylene and polypropylene resins. Of these, polyester, polyacrylate and polyamide are more preferable.

The above resin can be easily spun together with the heat-accumulative material or composition of the present invention into the heat-accumulative composite fiber of core/sheath structure.

The heat-accumulative composite fiber of the present invention can be produced by spinning the heat-accumulative material or composition and synthetic resin by a known extruder type spinning machine for composite materials.

Spinning temperature varies depending on type of the fiber material used, but is normally in a range of around 180 to 350°C.

Content of the heat-accumulative material in the heat-accumulative composite fiber is preferably 0.5 to 70% by weight, more preferably 1 to 50% by weight.

The heat-accumulative composite fiber may contain, as required, one or more additives, e.g., moisture absorber, humectant and antistatic agent so far as an additive does not harm its characteristics.

The cross-sectional shape of the heat-accumulative composite fiber is not limited. It may be circular or non-circular, e.g., triangular or square.

Production of the heat-accumulative composite fiber involves no problem resulting from its evaporation, because the heat-accumulative material or composition as the starting material has a sufficiently high molecular weight. Moreover, since it is resin, it can be kneaded into another material and made into fibers. Further, it is easily subjected

to processing such as continuous spinning and kneading. Therefore, the heat-accumulative composite fiber of the present invention is excellent in spinning characteristics and easily produced.

The heat-accumulative composite fiber, containing the heat-accumulative material in the core, generates the latent heat at the melting point of the heat-accumulative material. More specifically, it generates a latent heat preferably of 1 J/g or more, more preferably 5 J/g or more. If its latent heat is less than 1 J/g, it may not exhibit sufficient heat accumulative effect. Its latent heat is more preferably 1 J/g or more, still more preferably 5 J/g or more, more preferably in a temperature range of -10 to 80°C, still more preferably 0 to 50°C.

This characteristic allows the heat-accumulative composite fiber to fully exhibit the temperature-controlling function against changes of ambient temperature or the like.

Moreover, use of the heat-accumulative material or composition for the core allows the heat-accumulative material to be uniformly dispersed in the fiber, thereby controlling fluctuations of its tensile strength or the like. At the same time, use of the above synthetic resin for the sheath makes the fiber surface similar to that of the conventional synthetic fiber. Therefore, it is easily handled, because it can be processed by conventional methods for making cloth or knit, or dyeing.

[Heat-accumulative cloth]

The heat-accumulative cloth of the present invention is composed of the heat-accumulative fiber, partly or totally.

It can be structurally in the form of cloth, knit, non-woven fabric or the like.

The heat-accumulative composite fiber may be combined with another type of fiber.

The heat-accumulative composite fiber of the present invention and the heat-accumulative cloth of the present invention comprising the fiber has a temperature-controlling function, like the above heat-accumulative material or the like, and is suitable for controlling temperature against body temperature by direct or indirect contact.

The heat-accumulative composite fiber and heat-accumulative cloth member comprising the fiber can be suitably used for textile products having a temperature-controlling function, like the heat-accumulative material or the like.

[Molded article]

The molded article of the present invention is produced by forming the heat-accumulative material or composition into a shape. The molded articles include those produced by injection, blow, slash, calendering, extrusion, inflation, foaming or compression molding. The article can be molded by a known molding process.

The molded articles can be suitably used for various areas with their excellent heat-accumulative capacity, e.g., various energy-saving type parts and parts for preventing excessive heating or cooling, e.g., building materials (e.g., heat insulating boards, floor heating parts, temperature-retaining type toilet seats, and house walls, ceilings and floors); residential goods (e.g., heat-retaining tableware and bottles, furniture, bedclothes and beds); automobile parts (e.g., parts for air-conditioners, heat insulators, handles and shift knobs); electric/electronic appliance parts; heat-exchanger parts for TV sets and copiers; and heat transfer device parts (e.g., heat-retaining type transfer rolls and coolants for electronic parts).

The molded article of the present invention, comprises the composition incorporated with the heat-accumulative material. Thus when it comes into contact with an energy-containing object, its base melts at a low melting point so that it can control temperature rise of the base by the heat of fusion. When it is placed in a cold space, it generates the solidification heat to prevent temperature decrease.

The molded article and heat-accumulative composition of the present invention can exhibit the following effects, in addition to the above.

(1) They can be easily molded by the common plastic molding process, e.g., injection, blow, extrusion, calendering, foaming or compression molding.

(2) They comprise a high-molecular-weight heat-accumulative material to exhibit an excellent heat-accumulative function, because of limited evaporation of the heat-accumulative material.

(3) They can exhibit their heat-accumulative function in a daily service temperature range.

(4) Conventionally, for example, when a heat-accumulative material, e.g., paraffin, is used for floor heating, it should be contained in a container to prevent leakage. However, the heat-accumulative composition of the present invention can be molded into a plastic shape for a specific purpose unlike the conventional technique. Thus according to the present invention, various devices containing the heat-accumulative material have a simpler structure.

(5) They have a high cost merit, because they do not use an expensive material.

EXAMPLES

The present invention is described by EXAMPLES, which by no means limit the present invention.
The properties were determined in EXAMPLES by the following methods.

(1) Molecular weight: Molecular weight as polystyrene was determined by a GPC analyzer (JASCO's) with tetrahydrofuran (hereinafter abbreviated to THF) as a solvent.
(2) Melting point, freezing point and latent heat: These properties were determined by differential scanning calorimetry (Perkin Elmer Japan's DSC-7), where 3 mg of the sample was heated or cooled at 10°C/minute.
(3) Solution viscosity: Viscosity (unit: $mm^2/s$) of the 40% by weight toluene solution was determined at 50°C in accordance with JIS K-2283.
(4) Kinematic viscosity: Kinematic viscosity (unit: $mm^2/s$) of the molten sample was determined at 50°C in accordance with JIS K-2283.
(5) Weight loss: Weight loss of 3 mg of the sample maintained at 150°C for 1 hour was determined by a TG-DTA analyzer (Seiko Instrument's), where air was flown at 300 mL/minute.

[Methacrylate-based heat-accumulative materials]

EXAMPLE 1

Polystearyl methacrylate was synthesized by the following procedure.

(1) A 2L four-mouthed separable flask, equipped with a nitrogen supply tube, stirrer and reflux system, was charged with 400 g of stearyl methacrylate as a monomer and 700 mL of THF as a solvent.
(2) While nitrogen was fed into the flask, the content of the flask, put in a water bath kept at 70°C, was heated with slowly stirring to dissolve the monomer.
(3) After the monomer was dissolved, 0.1 g of azobisisobutylonitrile (hereinafter abbreviated to AIBN) as a polymerization initiator was added to the flask, and the stirring was then continued. Nitrogen was blown to such an extent that THF could be refluxed.
(4) In 1 hour, temperature of the water bath was adjusted in such a way to keep the flask inside at 70 to 75°C, at which the reaction process was allowed to proceed for 7 to 9 hours, to prepare a reaction solution.
(5) The resulting reaction solution was poured little by little into 4L of methanol with stirring, to precipitate a white solid. The precipitate was filtered, and dried by air and then under a vacuum to finally prepare a white crystal.

The white crystal was polystearyl methacrylate, as confirmed by NMR analysis. The analytical data are given in Table 1.

Table 1

| Chemical shift (ppm) | Number of protons | Relevant to: |
|---|---|---|
| 0.88 (triplet) | 3 | Stearyl $CH_3$ |
| 1.02 (broad) | 3 | Methacrylate $CH_3$ |
| 1.26 (broad) | 30 | Stearyl $CH_2 \times 15$ |
| 1.60 (broad) | 2 | Stearyl $CH_2$ |
| 1.79 (broad) | 2 | Methacrylate $CH_2$ |
| 3.91 (broad) | 2 | Stearyl $CH_2$-OC=O |

The white crystal had a weight-average molecular weight of 800,000.
Its various properties were measured. The results are given in Table 2.
The table also shows properties of octadecane and stearyl methacrylate for reference.

EXAMPLE 2

Polystearyl methacrylate was synthesized in the same manner as in EXAMPLE 1, except that THF as a solvent was replaced by toluene.

It had a weight-average molecular weight of 610,000.
Its various properties were measured. The results are given in Table 2.

EXAMPLE 3

Polystearyl methacrylate was synthesized in the same manner as in EXAMPLE 2, except that quantity of AIBN added was changed to 0.2 g.
It had a weight-average molecular weight of 330,000.
Its various properties were measured. The results are given in Table 2.

EXAMPLE 4

Polystearyl methacrylate was synthesized in the same manner as in EXAMPLE 2, except that quantity of AIBN added was changed to 1.0 g and temperature at which it was added was changed to 85°C.
It had a weight-average molecular weight of 124,000.
Its various properties were measured. The results are given in Table 2.

EXAMPLES 5 and 6

Polystearyl methacrylate was synthesized in the same manner as in EXAMPLE 2, except that quantity of AIBN added and temperature at which it was added were adjusted at given levels.
Each had a weight-average molecular weight of 200,000 (EXAMPLE 5) and 270,000 (EXAMPLE 6).
Their various properties were measured. The results are given in Table 2.

Table 2

| | Polymer prepared | Mw | A | Melting point (°C) | Freezing point (°C) | ΔT (°C) | Latent heat (J/g) | Solution viscosity | Weight loss |
|---|---|---|---|---|---|---|---|---|---|
| EXAMPLE 1 | Polystearyl methacrylate | 800,000 | 0.80 | 37 | 24 | 13 | 89 | 9,552 | 2% or less |
| EXAMPLE 2 | Polystearyl methacrylate | 610,000 | 0.80 | 35 | 24 | 11 | 75 | 3,421 | 2% or less |
| EXAMPLE 3 | Polystearyl methacrylate | 330,000 | 0.80 | 37 | 24 | 13 | 76 | 491.3 | 2% or less |
| EXAMPLE 4 | Polystearyl methacrylate | 124,000 | 0.80 | 37 | 24 | 13 | 79 | 120.6 | 2% or less |
| EXAMPLE 5 | Polystearyl methacrylate | 200,000 | 0.80 | 38 | 23 | 15 | 84 | 213.6 | 2% or less |
| EXAMPLE 6 | Polystearyl methacrylate | 270,000 | 0.80 | 39 | 24 | 15 | 77 | 331.7 | 2% or less |
| REFER-ENCE | Octadecane | 254*1 | — | 30 | 20 | 10 | 225 | 3.330*2 | 99% or more |
| REFER-ENCE | Stearyl methacrylate | 338*1 | — | 34 | 18 | 16 | 200 | 5.874*2 | 8% |

Mw: Weight-average molecular weight
ΔT: Melting point-freezing point
A: Z/(X+Y+Z)
*1: Molecular weight, *2: Kinematic viscosity

Octadecane and stearyl methacrylate as the reference compounds are of low molecular weight, and hence when molten, they are too low in viscosity to be practical. Moreover, octadecane is massively evaporated to lose weight significantly.

[Acrylate-based heat-accumulative materials]

EXAMPLE 7

Polystearyl acrylate was synthesized in the same manner as in EXAMPLE 2, except that stearyl acrylate was used as a monomer, quantity of the monomer added was decreased to one-fifth, and quantity of AIBN added was changed to 0.2 g.
It had a weight-average molecular weight of 220,000.
Its various properties were measured. The results are given in Table 4.

EXAMPLE 8

Polystearyl acrylate was synthesized in the same manner as in EXAMPLE 7, except that quantity of AIBN added was changed to 0.1 g.
It had a weight-average molecular weight of 700,000.
Its various properties were measured. The results are given in Table 4.

[Copolymer-based heat-accumulative materials]

EXAMPLE 9

Poly(stearyl methacrylate/2-hydroxyethyl methacrylate) copolymer (375 g) was synthesized in the same manner as in EXAMPLE 1, except that 356 g of stearyl methacrylate and 59 g of 2-hydroxyethyl methacrylate (molar ratio: 7/3) were used as the monomers.
The poly(stearyl methacrylate/2-hydroxyethyl methacrylate) was found to be a copolymer, as confirmed by NMR analysis. The analytical data are given in Table 3.

Table 3

| Chemical shift (ppm) | Number of protons | Relevant to: |
|---|---|---|
| 0.88 (triplet) | 2.1 | Stearyl $CH_3$ |
| 1.03 (broad) | 3 | Methacrylate $CH_3$ |
| 1.26 (broad) | 21 | Stearyl $CH_2\times15$ |
| 1.60 (broad) | 1.4 | Stearyl $CH_2$ |
| 1.85 (broad) | 2 | Methacrylate $CH_2$ |
| 3.84 (broad) | 0.6 | Hydroxyethyl $CH_2$-OH |
| 3.92 (broad) | 1.4 | Stearyl $CH_2$-OC=O |
| 4.11 (broad) | 0.6 | Hydroxyethyl $CH_2$-OC=O |

It had a weight-average molecular weight of 710,000.
Its various properties were measured. The results are given in Table 4.

EXAMPLE 10

Poly(stearyl methacrylate/2-hydroxyethyl methacrylate) copolymer was synthesized in the same manner as in EXAMPLE 5, except that the monomer molar ratio was changed from 7/3 to 9/1.
It had a weight-average molecular weight of 490,000.
Its various properties were measured. The results are given in Table 4.

Table 4

| | Polymer prepared | Mw | A | Melting point (°C) | Freezing point (°C) | ΔT (°C) | Latent heat (J/g) | Weight loss |
|---|---|---|---|---|---|---|---|---|
| EXAMPLE 7 | Polystearyl acrylate | 220,000 | 0.83 | 56 | 42 | 14 | 87 | 2% or less |
| EXAMPLE 8 | Polystearyl acrylate | 700,000 | 0.83 | 57 | 42 | 15 | 84 | 2% or less |
| EXAMPLE 9 | Poly(stearyl methacrylate/ 2-hydroxyethyl methacrylate) copolymer (7:3) | 710,000 | — | 31 | 19 | 12 | 71 | 2% or less |
| EXAMPLE 10 | Poly(stearyl methacrylate/ 2-hydroxyethyl methacrylate) copolymer (9:1) | 490,000 | — | 36 | 23 | 13 | 72 | 2% or less |

Mw: Weight-average molecular weight
ΔT: Melting point-freezing point
A: Z/(X+Y+Z)

[Acrylate-based heat-accumulative materials]

EXAMPLES 11 to 13

Polylauryl acrylate, polymyristyl acrylate and polypalmityl acrylate were synthesized in these examples in a manner similar to that for EXAMPLE 7 or 8 using lauryl acrylate, myristyl acrylate and palmityl acrylate, respectively, as the monomers.
Their various properties were measured. The results are given in Table 5.

[Methacrylate-based heat-accumulative materials]

EXAMPLES 14 to 16

Polymyristyl methacrylate, polypalmityl methacrylate and poly(palmityl/stearyl) methacrylate were synthesized in these examples in a manner similar to that for EXAMPLE 1 using myristyl methacrylate, palmityl methacrylate and a mixture of palmityl methacrylate and stearyl methacrylate, respectively, as the monomers.
Their various properties were measured. The results are given in Table 5.

[Vinyl ester-based heat-accumulative materials]

EXAMPLES 17 to 20

Polyvinyl laurate, polyvinyl myristate, polyvinyl palmitate and polyvinyl stearate were synthesized in these examples in a manner similar to that for EXAMPLE 1 using vinyl laurate, vinyl myristate, vinyl palmitate and vinyl stearate, respectively, as the monomers.
Their various properties were measured. The results are given in Table 5.

[Vinyl ether-based heat-accumulative material]

EXAMPLE 21

Polylauryl vinyl ether was prepared by a common cationic polymerization process using lauryl vinyl ether as the monomer, $BF_3$ ether complex as a catalyst and toluene as a solvent.
Its various properties were measured. The results are given in Table 5.

Table 5

| | Polymer prepared | Mw | Melting point (°C) | Freezing point (°C) | ΔT (°C) | Latent heat (J/g) | A | Weight loss |
|---|---|---|---|---|---|---|---|---|
| EX. 11 | Polylauryl acrylate | 160,000 | 12 | 0 | 12 | 55 | 0.77 | 2% or less |
| EX. 12 | Polymyristyl acrylate | 220,000 | 32 | 18 | 14 | 64 | 0.79 | 2% or less |
| EX. 13 | Polypalmityl acrylate | 200,000 | 43 | 30 | 13 | 76 | 0.81 | 2% or less |
| EX. 14 | Polymyristyl methacrylate | 280,000 | 10 | -5 | 15 | 52 | 0.75 | 2% or less |
| EX. 15 | Polypalmityl methacrylate | 330,000 | 22 | 8 | 14 | 60 | 0.77 | 2% or less |
| EX. 16 | Poly(palmityl/stearyl) Methacrylate (1/1) | 300,000 | 22-32 Two broad maxima | 8-18 | — | 60 | — | 2% or less |
| EX. 17 | Polyvinyl laurate | 80,000 | 16 | 3 | 13 | 62 | 0.81 | 2% or less |
| EX. 18 | Polyvinyl myristate | 50,000 | 28 | 15 | 13 | 80 | 0.83 | 2% or less |
| EX. 19 | Polyvinyl palmitate | 30,000 | 41 | 28 | 13 | 92 | 0.85 | 2% or less |
| EX. 20 | Polyvinyl stearate | 10,000 | 54 | 42 | 12 | 103 | 0.86 | 2% or less |
| EX. 21 | Polylauryl vinyl ether | 10,000 | 30 | 16 | 14 | 67 | 0.80 | 2% or less |

Mw: Weight-average molecular weight
A: Z/(X+Y+Z)
ΔT: Melting point-freezing point

Analytical Examples

The crystallized conditions of the side chains of the polymers prepared in EXAMPLES 2 and 5 were analyzed by an X-ray diffractometer (Rigaku's Geigerflex). The regular peaks relevant to the distance between the side chains and side chain length were confirmed.

Their degrees of crystallization were found by the peak separation method. The results are given in Table 6.

Table 6

| Analyzed Polymers | Mw | Melting point (°C) | Distance D between side chains (Å) | Side chain length L (Å) | Degree of crystallization (%) | Remarks |
|---|---|---|---|---|---|---|
| Polystearyl Methacrylate (prepared in EXAMPLE 2) | 610,000 | 35 | 4.2 | 29.4 | 59 | Before melting |
| | | | 4.1 | 30.4 | 45 | After melting, resolidified (after 30 minutes) |
| | | | 4.2 | 29.6 | 54 | After melting, resolidified (after 1 day) |
| Polystearyl Methacrylate (prepared in EXAMPLE 5) | 200,000 | 38 | 4.2 | 29.2 | 60 | Before melting |
| | | | 4.1 | 30.1 | 47 | After melting, resolidified (after 30 minutes) |
| | | | 4.2 | 29.4 | 55 | After melting, resolidified (after 1 day) |

Mw: Weight-average molecular weight

D: Distance between side chains

L: Side chain length

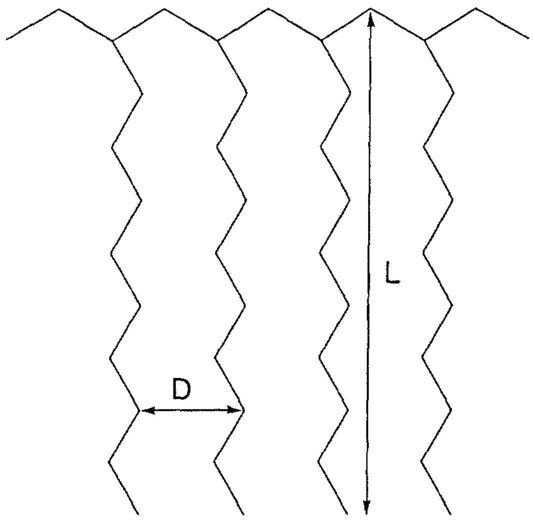


[Methacrylate-based heat-accumulative material]

EXAMPLE 22

Polystearyl methacrylate was synthesized by the following procedure.

A 2L four-mouthed separable flask, equipped with a nitrogen supply tube, stirrer and reflux system, was charged with 400 g of stearyl methacrylate as a monomer and 600 mL of toluene as a solvent.

While nitrogen was slowly fed, the content of the flask, put in a water bath kept at 65°C, was heated with stirring to dissolve stearyl methacrylate. After the monomer was dissolved, 1.0 g of AIBN as a polymerization initiator was added to the flask. The reaction was allowed to proceed for 8 hours, while bath temperature was adjusted to keep the reaction system at 75°C.

On completion of the reaction process, a reaction product was cooled to room temperature, and the product was added, with stirring, to 4L of methanol placed in a 5L beaker, to precipitate the polymer. The solution was stirred for 2 hours, and the precipitated polymer was separated by filtration and then dried by air, to prepare polystearyl methacrylate.

The resulting polymer had a weight-average molecular weight of 202,000, melting point of 38°C and latent heat of 84 J/g.

[Various heat-accumulative materials]

EXAMPLES 23 to 35

Various heat-accumulative materials, given in Table 7, were prepared in a manner similar to that for EXAMPLE 22.

Table 7

| | Polymer prepared | Mw | Melting point (°C) | Latent heat (J/g) |
|---|---|---|---|---|
| EX. 22 | Polystearyl methacrylate | 202,000 | 38 | 84 |
| EX. 23 | Polystearyl acrylate | 220,000 | 56 | 87 |
| EX. 24 | Poly(stearyl methacrylate/ 2-hydroxyethyl methacrylate) (7:3) | 710,000 | 31 | 71 |

Table 7 (continued)

| | Polymer prepared | Mw | Melting point (°C) | Latent heat (J/g) |
|---|---|---|---|---|
| EX. 25 | Polylauryl acrylate | 160,000 | 12 | 55 |
| EX. 26 | Polymyristyl acrylate | 220,000 | 32 | 64 |
| EX. 27 | Polypalmityl acrylate | 200,000 | 43 | 76 |
| EX. 28 | Polymyristyl methacrylate | 280,000 | 10 | 52 |
| EX. 29 | Polypalmityl methacrylate | 330,000 | 22 | 60 |
| EX. 30 | Poly(palmityl/stearyl) methacrylate (1/1) | 300,000 | 22-32<br>Two broad maxima | 60 |
| EX. 31 | Polyvinyl laurate | 80,000 | 16 | 62 |
| EX. 32 | Polyvinyl myristate | 50,000 | 28 | 80 |
| EX. 33 | Polyvinyl palmitate | 30,000 | 41 | 92 |
| EX. 34 | Polyvinyl stearate | 10,000 | 54 | 103 |
| EX. 35 | Polylauryl vinyl ether | 10,000 | 30 | 67 |
| Mw: Weight-average molecular weight | | | | |

[Cross-linked methacrylate-based heat-accumulative materials]

EXAMPLE 36

Cross-linked polystearyl methacrylate (degree of cross-linking: 1%) was synthesized by the following procedure.

(1) A 2L four-mouthed separable flask was equipped with a nitrogen supply tube, stirrer and reflux system.
(2) The flask was charged with 495 g of stearyl methacrylate (solid) as a monomer, 5 g of polyethylene glycol (1000) dimethacrylate (average molecular weight of the polyethylene glycol section: 1,000) and 300 mL of toluene as a solvent.
(3) While nitrogen was slowly introduced into the flask, the flask was put in an oil bath kept at 90°C and heated with slowly stirring (at about 200 rpm) to dissolve the solid.
(4) The resulting solution was heated, when it uniformly dissolved the solid, to a flask inside temperature of around 70°C. Then, 0.5 g of AIBN as a polymerization initiator was added, and the mixture was continuously stirred. Nitrogen flow rate was controlled in a range in which toluene could be refluxed.
(5) The flask content became gradually thickened when its temperature reached around 80°C, and pasty in around 20 minutes. Therefore, rotational speed of the stirrer was decreased to around 20 rpm, to prevent the content from ascending along the stirrer rod. It was stirred continuously for 3 hours after oil bath temperature was increased to 130°C while keeping its conditions unchanged.
(6) The reflux system and nitrogen supply tube for the flask were replaced by a vacuum distillation system, and pressure inside was slowly reduced while preventing clogging of the container by the expanded content, to remove toluene and unreacted light fractions. The pressure was reduced finally to around 2 torr.
(7) After about 2 hours, the content free of light fractions was transferred onto a Teflon$^R$ plate, and was roughly crushed, and dried by air and then under a vacuum to finally prepare 480 g of a white solid.

It was cross-linked polystearyl methacrylate, as confirmed by NMR analysis. The analytical data are given in Table 8.

Table 8

| Chemical shift (ppm) | Number of protons | Relevant to: |
|---|---|---|
| 0.88 (triplet) | 3 | Stearyl $CH_3$ |
| 1.02 (broad) | 3 | Methacrylate $CH_3$ |
| 1.26 (broad) | 30 | Stearyl $CH_2 \times 15$ |

Table 8 (continued)

| Chemical shift (ppm) | Number of protons | Relevant to: |
|---|---|---|
| 1.60 (broad) | 2 | Stearyl $CH_2$ |
| 1.79 (broad) | 2 | Methacrylate $CH_2$ |
| 3.91 (broad) | 2 | Stearyl $CH_2$-OC=O |

Its various properties were measured. The results are given in Table 9.
The table also shows properties of octadecane and stearyl methacrylate for reference.

EXAMPLE 37

Cross-linked polystearyl methacrylate (degree of cross-linking: 2%) was synthesized in the same manner as in EXAMPLE 36, except that quantities of stearyl methacrylate and polyethylene glycol dimethacrylate were changed to 490 and 10 g.
Its various properties were measured. The results are given in Table 9.

EXAMPLE 38

Cross-linked polystearyl methacrylate (degree of cross-linking: 3%) was synthesized in the same manner as in EXAMPLE 36, except that quantities of stearyl methacrylate and polyethylene glycol dimethacrylate were changed to 485 and 15 g.
Its various properties were measured. The results are given in Table 9.

EXAMPLE 39

Cross-linked polystearyl methacrylate (degree of cross-linking: 1%) was synthesized in the same manner as in EXAMPLE 36, except that quantities of stearyl methacrylate and ethylene glycol dimethacrylate as a cross-linking agent were changed to 495 and 5 g.
Its various properties were measured. The results are given in Table 9.

EXAMPLE 40

Cross-linked polystearyl methacrylate (degree of cross-linking: 2%) was synthesized in the same manner as in EXAMPLE 36, except that quantities of stearyl methacrylate and ethylene glycol dimethacrylate as a cross-linking agent were changed to 490 and 10 g.
Its various properties were measured. The results are given in Table 9.

EXAMPLE 41

Cross-linked polystearyl methacrylate (degree of cross-linking: 1%) was synthesized in the same manner as in EXAMPLE 36, except that toluene as the solvent was replaced by THF and reaction temperature was decreased to a THF reflux temperature. In this example, the solution was left at room temperature without removing the solvent by vacuum distillation. It turned into a white solid. It was roughly crushed, and put in hot water and stirred to remove THF from the solid. The solid no longer gave off an odor of THF, when the above procedure was repeated 5 times. It was dried by air and then under a vacuum to prepare 470 g of white, cross-linked powder.
Its various properties were measured. The results are given in Table 9.

Table 9

| | Cross-linked polymer prepared | Cross-linking agent (wt%) | Melting point (°C) | Freezing point (°C) | ΔT (°C) | Latent heat (J/g) | Weight loss |
|---|---|---|---|---|---|---|---|
| EXAMPLE 36 | Cross-linked polystearyl methacrylate*1 | 1 | 37 | 24 | 13 | 83 | 2% or less |
| EXAMPLE 37 | Cross-linked polystearyl methacrylate*1 | 2 | 37 | 24 | 13 | 84 | 2% or less |
| EXAMPLE 38 | Cross-linked polystearyl methacrylate*1 | 3 | 37 | 22 | 15 | 88 | 2% or less |
| EXAMPLE 39 | Cross-linked polystearyl Methacrylate*2 | 1 | 37 | 23 | 14 | 85 | 2% or less |
| EXAMPLE 40 | Cross-linked polystearyl Methacrylate*2 | 2 | 37 | 23 | 14 | 86 | 2% or less |
| EXAMPLE 41 | Cross-linked polystearyl methacrylate*1 | 1 | 37 | 23 | 14 | 85 | 2% or less |
| REFERENCE | Octadecane | — | 30 | 20 | 10 | 225 | 99% or more |
| REFERENCE | Stearyl methacrylate | — | 34 | 18 | 16 | 200 | 8% |

A: Z/(X+Y+Z); A=0.80

ΔT: Melting point-freezing point

*1: Polyethylene glycol (1000) dimethacrylate was used as a cross-linking agent

*2: Ethylene glycol dimethacrylate was used as a cross-linking agent

Octadecane and stearyl methacrylate as the reference compounds are of low molecular weight, and hence too low in viscosity, when molten, to be practical. Moreover, octadecane is massively evaporated to lose weight significantly.

[Cross-linked acrylate-based heat-accumulative materials]

EXAMPLE 42

Cross-linked polystearyl acrylate (degree of cross-linking: 1%) was synthesized in the same manner as in EXAMPLE 36, except that 495 g of stearyl acrylate was used as the monomer.
Its various properties were measured. The results are given in Table 12.

EXAMPLE 43

Cross-linked polystearyl acrylate (degree of cross-linking: 3%) was synthesized in the same manner as in EXAMPLE 36, except that 485 g of stearyl acrylate was used as the monomer.
Its various properties were measured. The results are given in Table 12.

[Cross-linked copolymer-based heat-accumulative materials]

EXAMPLE 44

Cross-linked poly(stearyl methacrylate/2-hydroxyethyl methacrylate) copolymer (480 g, degree of cross-linking: 1%) was synthesized in the same manner as in EXAMPLE 36, except that 425 g of stearyl methacrylate and 70 g of 2-hydroxyethyl methacrylate (molar ratio: 7/3) as the monomers, and 5 g of polyethylene glycol (1000) dimethacrylate as a cross-linking agent were used.
The poly(stearyl methacrylate/2-hydroxyethyl methacrylate) was found to be a cross-linked copolymer, as confirmed by NMR analysis. The analytical data are given in Table 10.

Table 10

| Chemical shift (ppm) | Number of protons | Relevant to: |
|---|---|---|
| 0.88 (triplet) | 2.1 | Stearyl $CH_3$ |
| 1.03 (broad) | 3 | Methacrylate $CH_3$ |
| 1.26 (broad) | 21 | Stearyl $CH_2 \times 15$ |
| 1.60 (broad) | 1.4 | Stearyl $CH_2$ |
| 1.85 (broad) | 2 | Methacrylate $CH_2$ |
| 3.84 (broad) | 0.6 | Hydroxyethyl $CH_2$-OH |
| 3.92 (broad) | 1.4 | Stearyl $CH_2$-OC=O |
| 4.11 (broad) | 0.6 | Hydroxyethyl $CH_2$-OC=O |

Its various properties were measured. The results are given in Table 12.

EXAMPLE 45

Cross-linked poly(stearyl methacrylate/2-hydroxyethyl methacrylate) copolymer (480 g, degree of cross-linking: 1%) was synthesized in the same manner as in EXAMPLE 44, except that the monomer molar ratio was changed from 7/3 to 9/1.
The poly(stearyl methacrylate/2-hydroxyethyl methacrylate) was found to be a cross-linked copolymer, as confirmed by NMR analysis. The analytical data are given in Table 11.

Table 11

| Chemical shift (ppm) | Number of protons | Relevant to: |
|---|---|---|
| 0.88 (triplet) | 2.7 | Stearyl $CH_3$ |

Table 11 (continued)

| Chemical shift (ppm) | Number of protons | Relevant to: |
|---|---|---|
| 1.03 (broad) | 3 | Methacrylate $CH_3$ |
| 1.26 (broad) | 27 | Stearyl $CH_2 \times 15$ |
| 1.60 (broad) | 1.8 | Stearyl $CH_2$ |
| 1.85 (broad) | 2 | Methacrylate $CH_2$ |
| 3.84 (broad) | 0.2 | Hydroxyethyl $CH_2$-OH |
| 3.92 (broad) | 1.8 | Stearyl $CH_2$-OC=O |
| 4.11 (broad) | 0.2 | Hydroxyethyl $CH_2$-OC=O |

Its various properties were measured. The results are given in Table 12.

Table 12

| | Cross-linked polymer prepared* | Cross-linking agent (wt%) | Melting point (°C) | Freezing point (°C) | ΔT (°C) | Latent heat (J/g) | A | Weight loss |
|---|---|---|---|---|---|---|---|---|
| EXAMPLE 42 | Cross-linked polystearyl acrylate | 1 | 56 | 42 | 14 | 83 | 0.83 | 2% or less |
| EXAMPLE 43 | Cross-linked polystearyl acrylate | 3 | 57 | 42 | 15 | 84 | 0.83 | 2% or less |
| EXAMPLE 44 | Cross-linked poly(stearyl methacrylate/2-hydroxyethyl methacrylate) copolymer (7:3) | 1 | 31 | 19 | 12 | 71 | — | 2% or less |
| EXAMPLE 45 | Cross-linked poly(stearyl methacrylate/2-hydroxyethyl methacrylate) copolymer (9:1) | 1 | 36 | 23 | 13 | 72 | — | 2% or less |

*: Polyethylene glycol (1000) dimethacrylate was used as a cross-linking agent
A: Z/(X+Y+Z)
ΔT: Melting point-freezing point

[Cross-linked acrylate-based heat-accumulative materials]

EXAMPLES 46 to 48

Cross-linked polylauryl acrylate, polymyristyl acrylate and polypalmityl acrylate (degree of cross-linking: 1% for all of these polymers) were synthesized in these examples in a manner similar to that for EXAMPLE 36 using lauryl acrylate, myristyl acrylate and palmityl acrylate, respectively, as the monomers.

Their various properties were measured. The results are given in Table 13.

[Cross-linked methacrylate-based heat-accumulative materials]

EXAMPLES 49 to 51

Cross-linked polymyristyl methacrylate, polypalmityl methacrylate and poly(palmityl/stearyl) methacrylate (degree of cross-linking: 1% for all of these polymers) were synthesized in these examples in a manner similar to that for EXAMPLE 36 using myristyl methacrylate, palmityl methacrylate and a mixture of palmityl methacrylate and stearyl methacrylate, respectively, as the monomers.

Their various properties were measured. The results are given in Table 13.

[Cross-linked vinyl ester-based heat-accumulative materials]

EXAMPLES 52 to 55

Cross-linked polyvinyl laurate, polyvinyl myristate, polyvinyl palmitate and polyvinyl stearate (degree of cross-linking: 1% for all of these polymers) were synthesized in these examples in a manner similar to that for EXAMPLE 36 using vinyl laurate, vinyl myristate, vinyl palmitate and vinyl stearate, respectively, as the monomers.

Their various properties were measured. The results are given in Table 13.

[Cross-linked vinyl ether-based heat-accumulative material]

EXAMPLE 56

Cross-linked polylauryl vinyl ether (degree of cross-linking: 1%) was prepared by a common cationic polymerization process using lauryl vinyl ether as the monomer, polyethylene glycol (1000) dimethacrylate as a cross-linking agent, $BF_3$ ether complex as a catalyst and toluene as a solvent.

Its various properties were measured. The results are given in Table 13.

Table 13

| | Cross-linked polymer prepared* | Melting point (°C) | Freezing point (°C) | ΔT (°C) | Latent heat (J/g) | A | Weight loss |
|---|---|---|---|---|---|---|---|
| EXAMPLE 46 | Cross-linked polylauryl acrylate | 12 | 0 | 12 | 55 | 0.77 | 2% or less |
| EXAMPLE 47 | Cross-linked polymyristyl acrylate | 32 | 18 | 14 | 64 | 0.79 | 2% or less |
| EXAMPLE 48 | Cross-linked polypalmityl acrylate | 43 | 30 | 13 | 76 | 0.81 | 2% or less |
| EXAMPLE 49 | Cross-linked polymyristyl methacrylate | 10 | -5 | 15 | 52 | 0.75 | 2% or less |
| EXAMPLE 50 | Cross-linked polypalmityl methacrylate | 22 | 8 | 14 | 60 | 0.77 | 2% or less |
| EXAMPLE 51 | Cross-linked poly-(palmityl/stearyl) methacrylate (1/1) | 22-32 Two broad maxima | 8-18 | — | 60 | — | 2% or less |
| EXAMPLE 52 | Cross-linked polyvinyl laurate | 16 | 3 | 13 | 62 | 0.81 | 2% or less |
| EXAMPLE 53 | Cross-linked polyvinyl myristate | 28 | 15 | 13 | 80 | 0.83 | 2% or less |
| EXAMPLE 54 | Cross-linked polyvinyl palmitate | 41 | 28 | 13 | 92 | 0.85 | 2% or less |
| EXAMPLE 55 | Cross-linked polyvinyl stearate | 54 | 42 | 12 | 103 | 0.86 | 2% or less |
| EXAMPLE 56 | Cross-linked polylauryl vinyl ether | 30 | 16 | 14 | 67 | 0.80 | 2% or less |

*: Polyethylene glycol (1000) dimethacrylate (1 wt%) was used as a cross-linking agent
A: Z/(X+Y+Z)
ΔT: Melting point-freezing point

Analytical Examples

The crystallized conditions of the side chains of the cross-linked polymers prepared in EXAMPLES 36, 38, 44 and 45 were analyzed by an X-ray diffractometer (Rigaku's Geigerflex). The regular peaks relevant to the distance between the side chains and side chain length were confirmed.

Their degrees of crystallization were found by the peak separation method. The results are given in Table 14.

Table 14

| Analyzed cross-linked polymers | Cross-linking agent (wt%) | Distance D between side chains (Å) | Side chain length L (Å) | Degree of crystallization (%) |
|---|---|---|---|---|
| Cross-linked polystearyl methacrylate (prepared in EXAMPLE 36) | 1 | 4.2 | 30.2 | 62 |
| Cross-linked polystearyl methacrylate (prepared in EXAMPLE 38) | 3 | 4.1 | 31.1 | 55 |
| Cross-linked poly(stearyl methacrylate/2-hydroxyethyl methacrylate) copolymer (7:3) (prepared in EXAMPLE 44) | 1 | 4.2 | 35.0 | 20 |
| Cross-linked poly(stearyl methacrylate/2-hydroxyethyl methacrylate) copolymer (9:1) (prepared in EXAMPLE 45) | 1 | 4.2 | 32.4 | 45 |

D: Distance between side chains
L: Side chain length

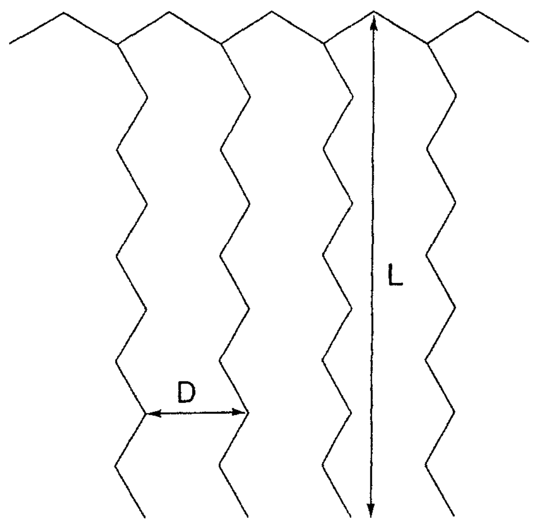


[Methacrylate-based heat-accumulative material]

EXAMPLE 57

Polystearyl methacrylate was synthesized by the following procedure.
A 3L four-mouthed separable flask, equipped with a nitrogen supply tube, stirrer and reflux system, was charged with 800 g of stearyl methacrylate as a monomer and 1.2 L of THF as a solvent.
While nitrogen was slowly introduced into the flask, the content of the flask, put in a water bath kept at 65°C, was heated with stirring for about 15 minutes to dissolve stearyl methacrylate. After the monomer was dissolved, 1.5 g of AIBN as a polymerization initiator was added to the flask. The reaction was allowed to proceed for 8 hours, while bath temperature was adjusted to keep the reaction system at 75°C.
On completion of the reaction process, a reaction product was cooled to room temperature, and the product was added, with stirring, to 4L of methanol placed in a 5L beaker, to precipitate the polymer. The solution was stirred for 2 hours, and the precipitated polymer was separated by filtration and then dried by air, to prepare polystearyl methacrylate.
The polymer was analyzed.
It had a weight-average molecular weight of 202,000, melting point of 38°C, freezing point of 24°C and latent heat of 84 J/g.

[Cross-linked methacrylate-based heat-accumulative materials]

EXAMPLE 58

Cross-linked polystearyl methacrylate (degree of cross-linking: 1%) was synthesized by the following procedure.

(1) A 2L four-mouthed separable flask was equipped with a nitrogen supply tube, stirrer and reflux system.
(2) The flask was charged with 743 g of stearyl methacrylate (solid) as a monomer, 7.5 g of polyethylene glycol (1000) dimethacrylate (average molecular weight of the polyethylene glycol section: 1,000) and 450 mL of toluene as a solvent.
(3) While nitrogen was slowly introduced into the flask, the flask was put in an oil bath kept at 90°C and heated with slowly stirring (at about 200 rpm) to dissolve the solid.
(4) The resulting solution was heated, when it uniformly dissolved the solid, to a flask inside temperature of around 70°C. Then, 0.75 g of AIBN as a polymerization initiator was added, and the mixture was continuously stirred.

Nitrogen flow rate was controlled in a range in which toluene could be refluxed.
(5) The flask content became gradually thickened when its temperature reached around 80°C, and pasty in around 20 minutes. Therefore, rotational speed of the stirrer was decreased to around 20 rpm, to prevent the content from ascending along the stirrer rod. It was stirred continuously for 3 hours after oil bath temperature was increased to 130°C while keeping its conditions unchanged.
(6) The reflux system and nitrogen supply tube for the flask were replaced by a vacuum distillation system, and pressure inside was slowly reduced while preventing clogging of the container by the expanded content, to remove toluene and unreacted light fractions. The pressure was reduced finally to around 2torr.
(7) After about 2 hours, the content free of light fractions was transferred onto a Teflon$^R$ plate, and was roughly crushed, and dried by air and then under a vacuum to finally prepare 720 g of a white solid.

It was cross-linked polystearyl methacrylate (degree of cross-linking: 1%), as confirmed by NMR analysis. Two batches of this cross-linked polymer were prepared.
The cross-linked polymer was analyzed.
It had a melting point of 37°C, freezing point of 24°C, ΔT (melting point-freezing point) of 13°C, latent heat of 83 J/g and weight loss of 2% or less.

[Sheets of heat-accumulative material]

EXAMPLES 59 to 72

Each of the heat-accumulative materials prepared in EXAMPLES 22 to 35 was spread by a coater and dried at 80°C on a releasing paper, and the releasing paper was separated to form a 100-μm-thick sheet.
Their melting points and latent heats are given in Table 15.

Table 15

| | Polymer used | Mw | Melting point (°C) | Latent heat (J/g) |
|---|---|---|---|---|
| EX. 59 | Polystearyl methacrylate | 202,000 | 38 | 84 |
| EX. 60 | Polystearyl acrylate | 220,000 | 56 | 87 |
| EX. 61 | Poly(stearyl methacrylate/ 2-hydroxyethyl methacrylate) (7:3) | 710,000 | 31 | 71 |
| EX. 62 | Polylauryl acrylate | 160,000 | 12 | 55 |
| EX. 63 | Polymyristyl acrylate | 220,000 | 32 | 64 |
| EX. 64 | Polypalmityl acrylate | 200,000 | 43 | 76 |
| EX. 65 | Polymyristyl methacrylate | 280,000 | 10 | 52 |
| EX. 66 | Polypalmityl methacrylate | 330,000 | 22 | 60 |
| EX. 67 | Poly(palmityl/stearyl) methacrylate (1/1) | 300,000 | 22-32<br>Two broad maxima | 60 |
| EX. 68 | Polyvinyl laurate | 80,000 | 16 | 62 |
| EX. 69 | Polyvinyl myristate | 50,000 | 28 | 80 |
| EX. 70 | Polyvinyl palmitate | 30,000 | 41 | 92 |
| EX. 71 | Polyvinyl stearate | 10,000 | 54 | 103 |
| EX. 72 | Polylauryl vinyl ether | 10,000 | 30 | 67 |
| Mw: Weight-average molecular weight | | | | |

[Sheets of heat-accumulative composition]

EXAMPLE 73

A mixed resin solution of 1,000 g of a moisture-permeable polyurethane resin solution (Dainichiseika Industry Co., Ltd., HaimulenY-237: solid content: 25% by weight) and 583 g of the heat-accumulative material prepared in

EXAMPLE 22 was spread by a coater and dried at 80°C on a releasing paper, and the releasing paper was separated to form a 100-µm-thick sheet.

EXAMPLE 74

A 100-µm-thick sheet was prepared in the same manner as in EXAMPLE 73, except that 250 g of the heat-accumulative material prepared in EXAMPLE 22 was used for the mixed resin solution.

COMPARATIVE EXAMPLE 1

A 100-µm-thick sheet was prepared in the same manner as in EXAMPLE 73, except that no heat-accumulative material was used.

COMPARATIVE EXAMPLE 2

A 100-µm-thick sheet was prepared in the same manner as in EXAMPLE 74, except that the heat-accumulative material prepared in EXAMPLE 22 was replaced by stearyl methacrylate dimmer.

[Laminate: Fiber]

EXAMPLE 75

The resin solution prepared in EXAMPLE 74 was spread on a base of mixed fibers of Nylon and Tetoron (50/50) by a wet coater. The coated base was passed through a solidification/water-washing tank and dried at 140°C to prepare a 100-µm-thick laminate.

The sheets prepared in EXAMPLES 73 and 74 and COMPARATIVE EXAMPLE 1, and laminate prepared in EXAMPLE 75 were measured for their latent heats at 38°C (melting point of the heat-accumulative material). The results are given in Table 16.

The sheet prepared in COMPARATIVE EXAMPLE 2 was too sticky to be suitable for various products, because of dissolution/elution of the stearyl methacrylate dimmer component.

Table 16

| | Content of heat-accumulative material in heat-accumulative composition (wt%) | Latent heat (J/g) |
|---|---|---|
| EXAMPLE 73 | 70 | 58 |
| EXAMPLE 74 | 50 | 42 |
| EXAMPLE 75 | 50 | 42 |
| COMPARATIVE EXAMPLE 1 | 0 | 0 |

[Laminates: Urethane resin]

EXAMPLE 76

The laminate 10, illustrated in Fig. 1 (a), was prepared by the following procedure.

A polyester-based urethane resin solution (Dainichiseika Industry Co., Ltd., Rezamin ME-3612LP) was spread by a bar coater on the releasing paper 11 and dried at 80°C, to prepare the 10-µm-thick urethane layer 12 (Layer A).

A coating solution was prepared by mixing 1000 g of a two-liquid urethane resin containing solids at 60% (Dainichiseika Industry Co., Ltd., Binder Rezamin UD-660SA), 120 g of a crosslinking agent (Dainichiseika Industry Co., Ltd., Rezamin UD-102), 100 g of a promoter (Dainichiseika Industry Co., Ltd., Rezamin UD-102), 600 g of toluene and 600 g of the heat-accumulative material prepared in EXAMPLE 22.

The coating solution was spread by a bar coater on Layer A and dried at 100°C, to prepare the 100-µm-thick urethane/heat-accumulative material layer 13 (heat-accumulative composition layer).

The above layer was laminated with the non-woven polyester fabric 14 at 120°C by a press, and then the releasing paper 11 was separated, to prepare the laminate 10.

EXAMPLE 77

The laminate 20 illustrated in Fig. 1 (b) was prepared by the following procedure.

A mixture of 1,000 g of toluene and 400 g of the heat-accumulative material prepared in EXAMPLE 22 as a coating solution was spread on the urethane layer 22 formed on the releasing paper 21 by a bar coater and dried at 80°C, to prepare the 70-μm-thick heat-accumulative material layer 23, in a manner similar to that for EXAMPLE 76.

The coating solution prepared in EXAMPLE 76, mixing 1000 g of a two-liquid urethane resin, 120 g of a crosslinking agent and 100 g of a promoter, was spread by a bar coater on the above heat-accumulative material layer 23 and dried at 100°C, to prepare the 10-μm-thick urethane binder layer 24.

The above layer was laminated with the non-woven polyester fabric 25 at 120°C by a press, and then the releasing paper 21 was separated, to prepare the laminate 20.

Various laminate structures comprising a urethane resin can be formed, in addition to those prepared in EXAMPLES 76 and 77. For example, the laminate 30 illustrated in Fig. 1 (C) can be formed. This laminate 30 comprises the urethane layer 31, urethane/heat-accumulative material layer (heat-accumulative composition layer) 32, urethane layer 33, binder layer 34, and paper or cloth layer 35. It has a still higher heat-accumulative effect, because urethane in the urethane/heat-accumulative material layer 32 and/or urethane layer 33 is foamed.

COMPARATIVE EXAMPLE 3

The laminate was prepared in the same manner as in EXAMPLE 76, except that no heat-accumulative material was incorporated.

Each of the laminates prepared in EXAMPLES 76 and 77 and COMPARATIVE EXAMPLE 3 was folded in four to contain thermocouples inside, and subjected to environmental temperature changing from 25°C to 40°C and then to 5°C, to measure temperature changes inside. The results are given in Fig. 2.

As understood from the graph in Fig. 2, each of the laminates prepared in EXAMPLES 76 and 77 was less sensitive to environmental temperature changes than that prepared in COMPARATIVE EXAMPLE 3.

[Laminates: Leathers]

EXAMPLE 78

A mixture of 50% by weight of a soft vinyl chloride resin (Shin-Etsu Polymer, Plasticizer: 38% by weight, Average degree of polymerization: 3000) and 50% by weight of the heat-accumulative material prepared in EXAMPLE 22 was prepared by kneading under heating at 160°C. It was formed into a 400-μm-thick sheet, and put on a polyester/rayon (50/50) cloth via a urethane-based binder, to prepare a vinyl chloride leather (PVC leather).

COMPARATIVE EXAMPLE 4

A laminate was prepared in the same manner as in EXAMPLE 78, except that no heat-accumulative material was incorporated.

Each of the sheets prepared in EXAMPLE 78 and COMPARATIVE EXAMPLE 4 was measured for its latent heat at 38°C (melting point of the heat-accumulative material). The results are given in Table 17.

Table 17

| | Latent heat (J/g) |
|---|---|
| EXAMPLE 78 | 45 |
| COMPARATIVE EXAMPLE 4 | 0 |

[Heat-accumulative composite fibers and cloths]

EXAMPLES 79 to 92

Each of the heat-accumulative materials prepared in EXAMPLE 22 to 25 and Nylon 6 were spun by an extruder type composite spinning machine into the composite fibers. The spinning process was carried out to separately melt the heat-accumulative material and Nylon 6 and discharge them via mouth pieces in such a way to form the composite fibers of core-sheath structure, with the former serving as the core and the latter for the sheath. They were thermally

set by drawing rollers and wound to prepare the drawn fibers (heat-accumulative composite fibers), each 38 deniers in size and comprising 12 filaments. The fiber contained the heat-accumulative material at around 35% by weight.
The heat-accumulative composite fibers were wound around a urethane fiber to prepare single-covered type yarns, which were knitted by a circular knitting machine to prepare a knit (heat-accumulative cloth).

EXAMPLE 93

Polypropylene (IDEMITSU PP Y-2005GP) was incorporated with 30% by weight of the heat-accumulative material of polystearyl methacylate prepared in EXAMPLE 22, and kneaded at 230°C by a double-screw extruder (PCM-30, made by Ikegai Iron Works, Ltd.) under a molten condition to prepare the heat-accumulative composition.
The 40-denier/12-filament drawn heat-accumulative composite fibers were prepared in the same manner as in EXAMPLE 79, except that the above composition was used, and formed into a knit as the heat-accumulative cloth also in the same manner. The composite fiber contained polystearyl methacrylate at around 15% by weight.

EXAMPLE 94

A heat-accumulative composition was prepared in a manner similar to that for EXAMPLE 93 using Nylon 6 incorporated with 20% by weight of the heat-accumulative material of polystearyl methacylate prepared in EXAMPLE 22.
The 40-denier/12-filament drawn heat-accumulative composite fibers were prepared in the same manner as in EXAMPLE 93, except that the above composition was used, and formed into a knit as the heat-accumulative cloth also in the same manner. The composite fiber contained polystearyl methacrylate at around 10% by weight.

Example 95

Polyethylene terephthalate (PET) resin was incorporated with 20% by weight of the heat-accumulative material of polystearyl methacylate prepared in EXAMPLE 22, to prepare the heat-accumulative composition in a manner similar to that for EXAMPLE 93. The composition was formed into the 40-denier/12-filament drawn heat-accumulative composite fibers, with the composition serving as the core and PET resin as the sheath, in a manner similar to that for EXAMPLE 93, and then into a knit as the heat-accumulative cloth also in the same manner. The composite fiber contained polystearyl methacrylate at around 10% by weight.

COMPARATIVE EXAMPLE 5

The 38-denier/12-filament drawn heat-accumulative composite fibers were prepared in the same manner as in EXAMPLE 79, except that the heat-accumulative material used for the core was replaced by polypropylene, and formed into a knit as the heat-accumulative cloth also in the same manner.

COMPARATIVE EXAMPLE 6

The 70-denier/24-filament drawn heat-accumulative composite fibers were prepared in the same manner as in EXAMPLE 79, except that the heat-accumulative material used for the core was replaced by Nylon 6 as the same material used for the sheath, and formed into a knit as the heat-accumulative cloth also in the same manner.
Each of the cloth prepared in EXAMPLES and COMPARATIVE EXAMPLES was cut into a 10-cm-square shape, which was wound around thermocouples to prepare a test sample.
The test sample was allowed to stand in an atmosphere kept at 20°C, and then transferred in an atmosphere kept at 50°C, to measure time required for the sample to reach 40°C determined by the thermocouples to evaluate temperature-controlling function of each cloth . The results are given in Table 18.

Table 18

| | Required time (minutes) |
|---|---|
| EXAMPLE 79 | 1 8 |
| EXAMPLE 80 | 1 8 |
| EXAMPLE 81 | 1 6 |
| EXAMPLE 82 | 1 5 |

Table 18 (continued)

| | Required time (minutes) |
|---|---|
| EXAMPLE 83 | 1 6 |
| EXAMPLE 84 | 1 6 |
| EXAMPLE 85 | 1 5 |
| EXAMPLE 86 | 1 6 |
| EXAMPLE 87 | 1 6 |
| EXAMPLE 88 | 1 6 |
| EXAMPLE 89 | 1 8 |
| EXAMPLE 90 | 2 1 |
| EXAMPLE 91 | 2 3 |
| EXAMPLE 92 | 1 3 |
| EXAMPLE 93 | 1 2 |
| EXAMPLE 94 | 1 1 |
| EXAMPLE 95 | 1 2 |
| COMPARATIVE EXAMPLE 5 | 7 |
| COMPARATIVE EXAMPLE 6 | 6 |

[Injection-molded articles]

EXAMPLE 96

A mixture of 70 parts by weight of polypropylene resin (IDEMITSU PP J466H (trade name), made by Idemitsu Petrochemical Co., Ltd., melting point: 158°C, PP) and 30 parts by weight of the polystearyl methacylate prepared in EXAMPLE 57 by dry blending, and kneaded at 200°C by a 35mm-diameter extruder (made by Ikegai Iron Works, Ltd.) to prepare the heat-accumulative PP composition.

The PP composition was molded at 210°C into a 15-cm-square, 4-mm-thick flat plate by a 20-ton small-size injection molder. It was left in a constant-temperature tank kept at 50°C for 0.5 hour and then in a constant-temperature tank kept at 5°C for 0.5 hour, to follow temperature changes of the base material by a surface thermometer, and thereby to evaluate its heat-accumulative capacity. The results are given in Fig. 3. The composition was also molded into a container, to evaluate temperature level in the air layer and water layer respectively, when it was filled with air and water. The results are given in Fig. 4 and Fig. 5, respectively. The result of evaluating heat-accumulative capacity of the composition is given in Table 19.

COMPARATIVE EXAMPLE 7

A 4-mm-thick flat plate was prepared in the same manner as in EXAMPLE 96, except that no polystearyl methacrylate was used, to evaluate its heat-accumulative capacity. The results are given in Fig. 3. The composition was also molded into a container, to evaluate temperature level in the air layer and water layer, when it was filled with air and water respectively. The results are given in Fig. 4 and Fig. 5, respectively.

[Blow molded article]

EXAMPLE 97

A mixture of 70parts by weight of high-density polyethylene resin (IDEMITSU HD 520MB (trade name), made by Idemitsu Petrochemical Co., Ltd., melting point: 130°C, HDPE) and 30parts by weight of the polystearyl methacylate prepared in EXAMPLE 57 by dry blending, and kneaded at 180°C by a 35mm-diameter extruder (made by Ikegai Iron Works, Ltd.) to prepare the heat-accumulative HDPE composition.

The HDPE composition was molded at 180°C into a 200 cc multi-layered hollow bottle, with a 2-mm-thick

intermediate layer of the HDPE composition and 1-mm-thick inner and outer layers of the HDPE by a two kind-three layer small-size blow molder, to evaluate its heat-accumulative capacity. The result is given in Table 19.

[Compression-molded article]

EXAMPLE 98

A compression-molded article of the heat-accumulative PP composition prepared in EXAMPLE 96 was prepared by a process where 45 g of the PP composition, held by a 1-mm-thick, 20-cm-square frame, was pre-heated at 200°C for 10 minutes, degassed, pressed at 16 MPaG for 2 minutes, and cooled at 10 MPa by a cooling press operating at room temperature. In this process, a commonly used PET film was used for mold releasing, and 1-mm-thick aluminum plates were put on the upper and lower sides of the frame. The result of evaluating heat-accumulative capacity of the article is given in Table 19.

EXAMPLE 99

A heat-accumulative PP composition was prepared in the same manner as in EXAMPLE 96, except that the polystearyl methacrylate prepared in EXAMPLE 57 was replaced by the cross-linked polystearyl methacrylate prepared in EXAMPLE 58.

A compression-molded article of the above heat-accumulative PP composition was prepared in the same manner as in EXAMPLE 98. The evaluation result of its heat-accumulative capacity is given in Table 19.

[Expansion-molded articles]

EXAMPLE 100

A heat-accumulative urethane foam containing 30% by weight of heat-accumulative component was prepared by the common procedure using the polystearyl methacrylate prepared in EXAMPLE 57, polyol component, isocyanate component, foaming agent, catalyst and cell stabilizer, to evaluate its heat-accumulative capacity. Its composition is given below. The evaluation result is given in Table 19.

Polyol component: Dow Polyurethane's #3000 (trade name), 100 parts by weight
Isocyanate component: Dow Polyurethane's T-80 (trade name), 40 parts by weight
Foaming agent: Water, 2.9 parts by weight
Amine-based catalyst: Air Products' 33LV (trade name), 0.3 parts by weight
Amine-based catalyst: Air Products' AT33 (trade name), 0.3 parts by weight
Tin-based catalyst: Nitto Kasei's T-9 (trade name), 0.3 parts by weight
Cell stabilizer: Japan Uniker's L6202 (trade name), 0.3 parts by weight
Heat-accumulative material: Polystearyl methacrylate, 62 parts by weight

[Extrusion-molded article]

EXAMPLE 101

The heat-accumulative PP composition prepared in EXAMPLE 96 was molded by a 40mm-diameter small-size extruder for multi-layered articles, to prepare the 3-layered article with the heat-accumulative layer at the center, which contained the heat-accumulative component at 30% by weight, to evaluate its heat-accumulative capacity. The evaluation result is given in Table 19.

Table 19

| | Content of heat-accumulative material in molded article (wt%) | Latent heat (J/g) |
|---|---|---|
| EXAMPLE 96 | 30 | 20 |
| EXAMPLE 97 | 15 | 11 |
| EXAMPLE 98 | 30 | 23 |
| EXAMPLE 99 | 30 | 22 |

Table 19 (continued)

| | Content of heat-accumulative material in molded article (wt%) | Latent heat (J/g) |
|---|---|---|
| EXAMPLE 100 | 30 | 21 |
| EXAMPLE 101 | 30 | 20 |

Industrial Applicability

The present invention can provide a heat-accumulative material, heat-accumulative composition, and heat-accumulative film or sheet, laminate, molded article, composite fiber and cloth using the material or composition, which are easily produced and molded and have excellent heat-accumulative capacity.

## Claims

1. A heat-accumulative material for use around a body, comprising a polymer or oligomer whose melting point is from -10°C to 100°C and latent heat is at least 30 J/g.

2. The heat-accumulative material according to claim 1, wherein the melting point is from -10°C to 80°C.

3. The heat-accumulative material according to claim 1, wherein difference between the melting point and a solidifying point of the polymer or oligomer is at most 15°C.

4. The heat-accumulative material according to claim 1, wherein the melting point is from 0°C to 50°C.

5. The heat-accumulative material according to claim 4, wherein viscosity of a 40 wt.% toluene solution of the polymer or oligomer at 50°C is at least 100 $mm^2/s$.

6. The heat-accumulative material according to claim 1, wherein the latent heat is at least 50 J/g.

7. A heat-accumulative material which is a polymer or oligomer comprising crystalline units of formula (1), the unit comprising, as main components, a main chain section X, a bonding section Y and a side chain Z, side chains Z capable of being crystallized.

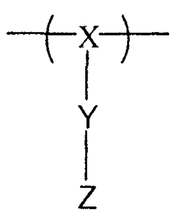

(1)

8. A heat-accumulative material which is a cross-linked polymer or oligomer comprising crystalline units of formula (1), the unit comprising, as main components, a main chain section X, a bonding section Y and a side chain Z, side chains Z capable of being crystallized.

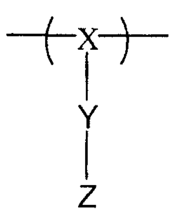

(1)

**9.** The heat-accumulative material according to claim 7 or 8, wherein weights of the main chain section X, the bonding section Y and side chain Z satisfy the following formula:

$$Z / (X + Y + Z) \geqq 0.75.$$

**10.** The heat-accumulative material according to claim 7 or 8, wherein the main chain section X is at least one selected from the group of

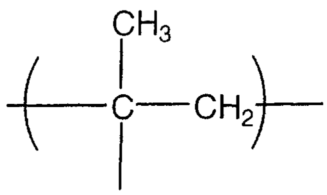 and 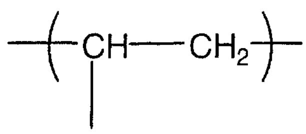 ,

the bonding section and side chain, Y-Z, is at least one selected from the group of -CO-O-R, -O-CO-R, -O-R and $-CH_2$-R, and

R is a hydrocarbon group having 9 or more carbon atoms.

**11.** The heat-accumulative material according to claim 10, wherein R is a straight hydrocarbon group having 9 or more carbon atoms.

**12.** The heat-accumulative material according to claim 7, which is polydocosyl methacrylate, polyheneicosyl methacrylate, polyeicosyl acrylate, polynonadesyl acrylate, polyheptadesyl acrylate, polypalmityl acrylate, polypentadesyl acrylate, polystearyl acrylate, polylauryl acrylate, polymyristyl acrylate, polymyristyl methacrylate, polypentadesyl methacrylate, polypalmityl methacrylate, polyheptadesyl methacrylate, polynonadesyl methacrylate, polyeicosyl methacrylate, polystearyl methacrylate, poly(palmityl/stearyl) methacrylate, polyvinyl laurate, polyvinyl myristate, polyvinyl palmitate, polyvinyl stearate, polylauryl vinyl ether, polymyristyl vinyl ether, polypalmityl vinyl ether or polystearyl vinyl ether.

**13.** The heat-accumulative material according to claim 7 or 8, the polymer or oligomer further comprising hydrophilic units.

**14.** The heat-accumulative material according to claim 13, the hydrophilic unit is represented by formula (2).

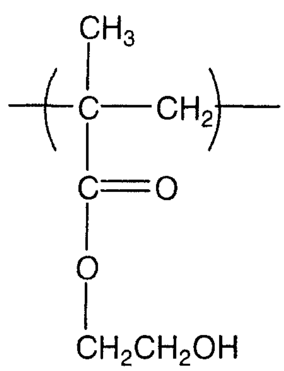

(2)

**15.** The heat-accumulative material according to claim 8 or 13, wherein a cross linking agent is 0.1 to 20 wt.% of monomers forming the crystalline units, or the crystalline units and the hydrophilic units.

**16.** A heat-accumulative composition comprising the heat-accumulative material of claim 1, 7 or 8 and a synthetic resin.

**17.** The heat-accumulative composition according to claim 16, wherein the synthetic resin is at least one selected from

the group of polyurethane, acrylic, polyamide, polyvinyl chloride, polypropylene, polyethylene, polystyrene, polyester, polycarbonate, ethylene/vinyl alcohol copolymer, thermoplastic elastomer, polyphenylene sulfide and ABS resins.

**18.** A heat-accumulative film or sheet comprising the material of claim 1, 7 or 8; or the composition of claim 16.

**19.** A heat-accumulative laminate comprising the film or sheet of claim 18 as one layer.

**20.** A heat-accumulative composite fiber comprising a core and a sheath;
the core comprising the material of claim 1, 7 or 8; or the composition of claim 16;
the sheath comprising a synthetic resin.

**21.** The heat-accumulative composite fiber according to claim 20, wherein the synthetic resin is at least one selected from the group of polyamide, polyester, polyurethane, ethylene/vinyl acetate copolymer, polyvinylidene chloride, polyvinyl chloride, acrylic, polyethylene and polypropylene resins.

**22.** A heat-accumulative cloth comprising the composite fiber of claim 20.

**23.** A heat-accumulative molded article comprising the material of claim 1, 7 or 8; or the composition of claim 16.

**24.** The heat-accumulative molded article according to claim 23, which is an energy-saving part or a part for preventing excessive heating or cooling.

**25.** The heat-accumulative molded article according to claim 23, which is a building material, residential good, automobile part, electric/electronic appliance part, heat-exchanger part or heat transfer device part.

Fig. 1

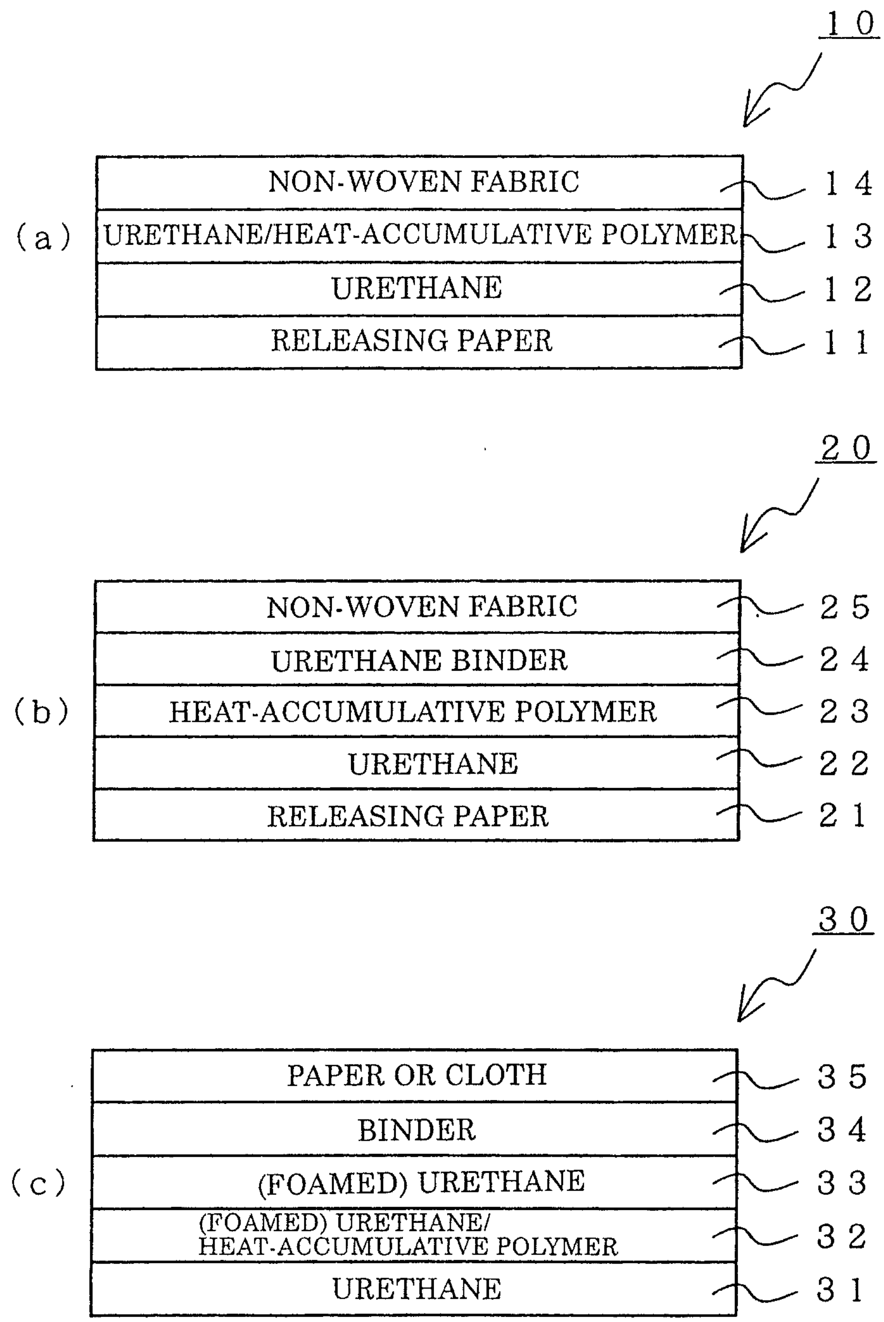
10
(a)
NON-WOVEN FABRIC
URETHANE/HEAT-ACCUMULATIVE POLYMER
URETHANE
RELEASING PAPER
14
13
12
11
20
(b)
NON-WOVEN FABRIC
URETHANE BINDER
HEAT-ACCUMULATIVE POLYMER
URETHANE
RELEASING PAPER
25
24
23
22
21
30
(c)
PAPER OR CLOTH
BINDER
(FOAMED) URETHANE
(FOAMED) URETHANE/
HEAT-ACCUMULATIVE POLYMER
URETHANE
35
34
33
32
31

Fig. 2

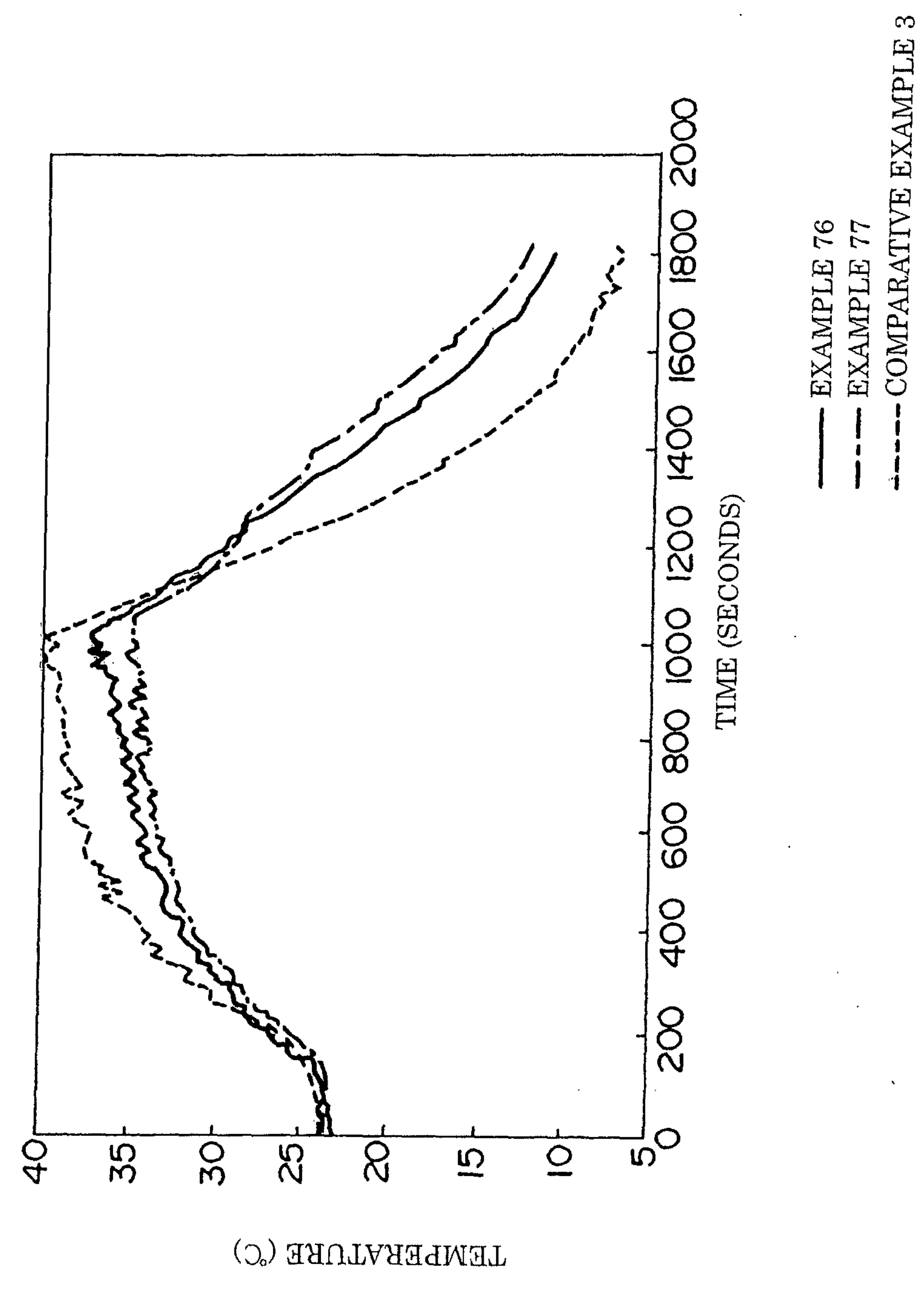
TEMPERATURE (°C)
40
35
30
25
20
15
10
5
0
200
400
600
800
1000
1200
1400
1600
1800
2000
TIME (SECONDS)
EXAMPLE 76
EXAMPLE 77
COMPARATIVE EXAMPLE 3

Fig. 3

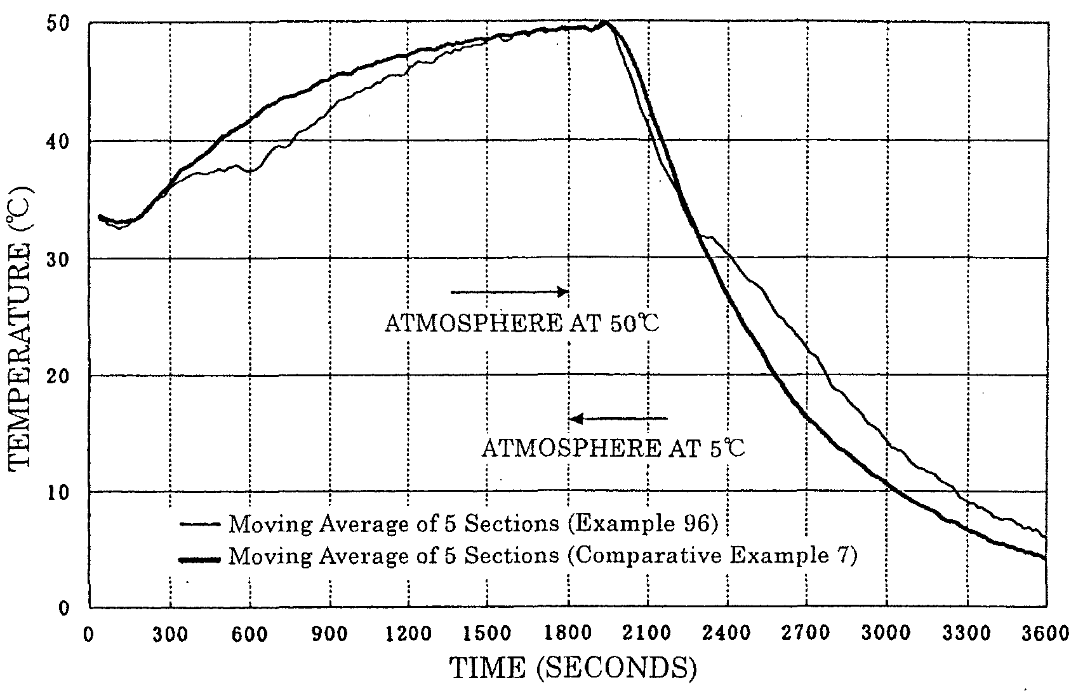
50
40
30
20
10
0
TEMPERATURE (℃)
0
300
600
900
1200
1500
1800
2100
2400
2700
3000
3300
3600
TIME (SECONDS)
ATMOSPHERE AT 50℃
ATMOSPHERE AT 5℃
Moving Average of 5 Sections (Example 96)
Moving Average of 5 Sections (Comparative Example 7)

Fig. 4

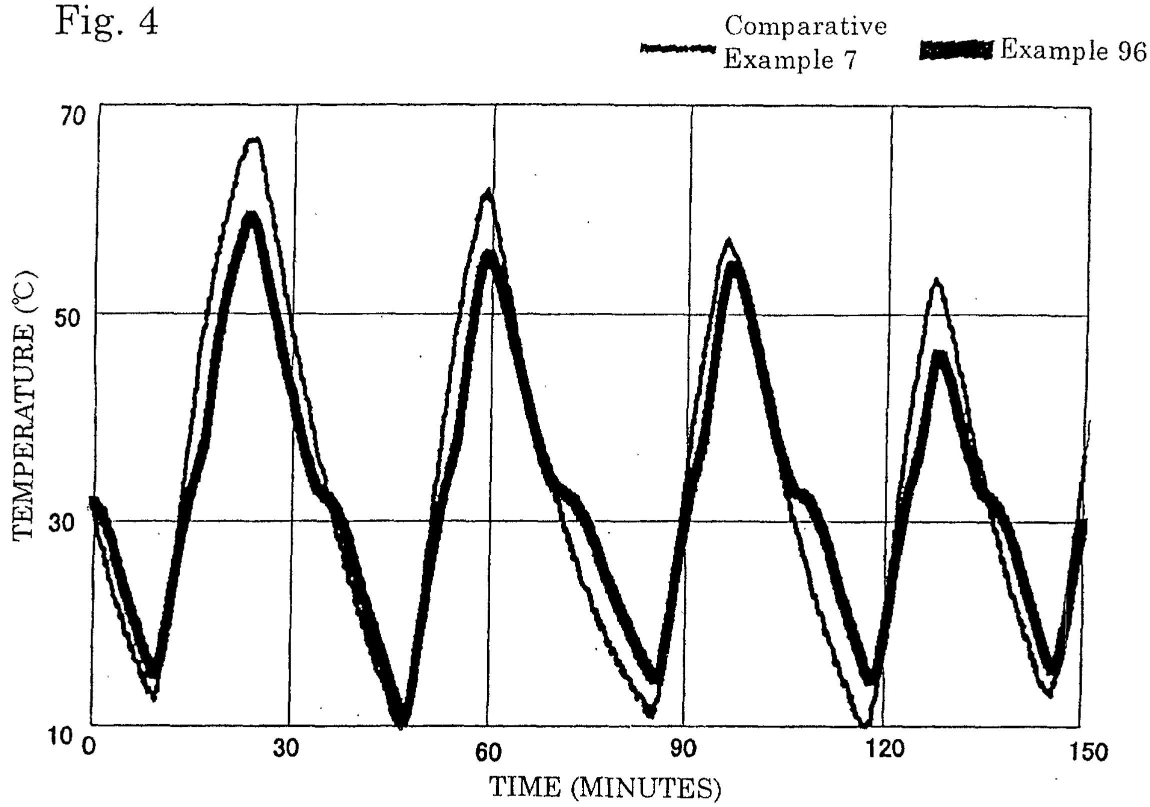
Comparative Example 7
Example 96
70
50
30
10
0
30
60
90
120
150
TEMPERATURE (℃)
TIME (MINUTES)

Fig. 5

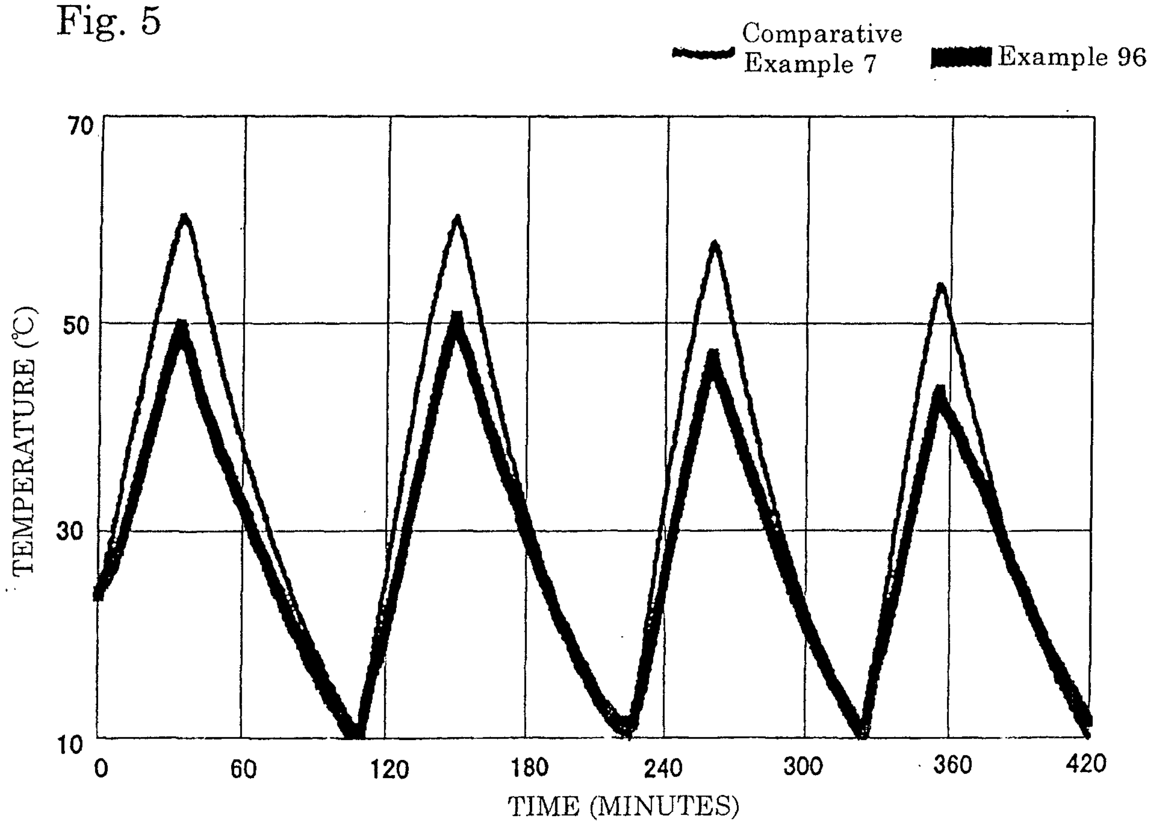
Comparative Example 7
Example 96
70
50
30
10
0
60
120
180
240
300
360
420
TEMPERATURE (℃)
TIME (MINUTES)

## INTERNATIONAL SEARCH REPORT

International application No.
PCT/JP03/02709

A. CLASSIFICATION OF SUBJECT MATTER
Int.Cl[7] C09K5/06, A61F7/08, A41D31/00

According to International Patent Classification (IPC) or to both national classification and IPC

B. FIELDS SEARCHED

Minimum documentation searched (classification system followed by classification symbols)
Int.Cl[7] C09K5/06, A61F7/08, A41D31/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 5-256549 A (Mitsubishi Cable Industries, Ltd.), 05 October, 1993 (05.10.93), Claim 1; examples 1 to 6 (Family: none) | 1-4 |
| A | JP 8-59956 A (Sumitomo Electric Industries, Ltd.), 05 March, 1996 (05.03.96), Claim 1; table 4 (Family: none) | 1-25 |
| A | JP 9-272861 A (Ube Industries, Ltd.), 21 October, 1997 (21.10.97), Claims 1 to 3; Par. Nos. [0031] to [0032] (Family: none) | 1-25 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 May, 2003 (19.05.03) | 03 June, 2003 (03.06.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/02709

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-279931 A (Mitsubishi Chemical Corp.), 20 October, 1998 (20.10.98), Claim 1 (Family: none) | 1-25 |

Form PCT/ISA/210 (continuation of second sheet) (July 1998)